Europäisches Patentamt

European Patent Office

Office européen des brevets

(19)

(11) Publication number: **0 018 020**
**B1**

(12) **EUROPEAN PATENT SPECIFICATION**

(45) Date of publication of patent specification: **14.03.84**

(51) Int. Cl.³: **B 29 D 7/22, A 41 B 13/02**

(21) Application number: **80200156.0**

(22) Date of filing: **26.02.80**

(54) Resilient plastic web exhibiting fiber-like properties and method for its manufacture.

(30) Priority: **05.03.79 US 17506**

(43) Date of publication of application:
**29.10.80 Bulletin 80/22**

(45) Publication of the grant of the patent:
**14.03.84 Bulletin 84/11**

(84) Designated Contracting States:
**AT BE CH DE FR GB IT LU NL SE**

(56) References cited:
**DE - A - 1 610 523**
**DE - A - 2 145 261**
**US - A - 2 809 392**
**US - A - 3 054 148**
**US - A - 3 814 101**
**US - A - 3 911 187**
**US - A - 3 929 135**
**US - A - 3 965 906**
**US - A - 3 989 867**
**US - A - 4 041 949**
**US - A - 4 041 951**
**US - A - 4 151 240**
**US - E - 23 910**

(73) Proprietor: **THE PROCTER & GAMBLE COMPANY**
**301 East Sixth Street**
**Cincinnati Ohio 45202 (US)**

(72) Inventor: **Radel, Clifford John**
**3973 Roswell Avenue**
**Cheviot Ohio 45211 (US)**
Inventor: **Thompson, Hugh Ansley**
**5777 Windermere Lane**
**Fairfield Ohio 45014 (US)**

(74) Representative: **Gibson, Tony Nicholas et al,**
**Procter & Gamble (NTC) Limited Whitley Road**
**Longbenton Newcastle upon Tyne NE12 9TS (GB)**

Courier Press, Leamington Spa, England.

Resilient plastic web exhibiting fiber-like properties and method for its manufacture

Technical field

The present invention has relation to resilient plastic webs exhibiting many of the three-dimensional, fiber-like properties and characteristics previously obtainable only in fibrous webs.

The present invention has further relation to resilient fluid-pervious plastic webs which exhibit a combination of desirable, but previously incompatible attributes of prior art fibrous webs and prior art plastic webs in a single structure without deleterious side effects.

The present invention has further relation to the provision of method for forming plastic webs exhibiting the aforementioned attributes.

Background art

It has long been known in the disposable absorbent bandage art that it is extremely desirable to construct absorptive devices, such as disposable diapers, sanitary napkins, and the like, presenting a dry surface feel to the user to improve wearing comfort and to minimize the development of undesirable skin conditions due to prolonged exposure to moisture absorbed within the bandage. One viable prior art solution to the aforementioned problem is disclosed in U.S. Patent 4,041,951 issued to Sanford on August 16, 1977 which teaches a preferred disposable diaper structure comprising a substantially planar, moisture absorbent layer disposed between a soft topsheet and a moisture-resistant backing sheet. The nonwoven fibrous topsheet preferably comprises an integral structure containing a multiplicity of depressed areas which intimately contact the uppermost surface of a substantially planar, moisture absorbent layer. The non-depressed areas of the topsheet contact the wearer's skin in use. In a particularly preferred embodiment, the nonwoven fibrous topsheet is comprised of a substantially hydrophobic material exhibiting wet resilience such that the topsheet tends to resume its substantially three-dimensional character upon removal of pressure applied against the topsheet by the body movements of the wearer. The nondepressed areas of the topsheet, which are of substantially the same density as the depressed areas, tend to isolate the wearer's skin from moisture contained within the moisture absorbent layer, thereby providing surface dryness and resistance to rewetting when the structure is temporarily subjected to pressure resulting from the wearer's body movements.

U.S. Patent 3,814,101 issued to Kozak on June 4, 1974, attacks the problem of a wet topsheet in a manner slightly different from the use of hydrophobic nonwoven materials. Kozak suggests a topsheet of a nonfibrous, hydrophobic film which is provided with a plurality of valvular slits which restrict the reverse flow of liquid from the absorbent element of the device.

U.S. patent 3,929,135 issued to Thompson on December 30, 1975, suggests a topsheet comprised of liquid-impermeable material, but provided with tapered capillaries, said capillaries having a base opening in the plane of the topsheet and an apex opening remote from the plane of the topsheet, said apex opening being in intimate contact with the absorbent pad utilized in the disposable absorbent bandage. The topsheet disclosed in the Thompson patent allows the free transfer of fluids from the body into the absorbent element of the device while inhibiting the reverse flow of these fluids, thereby providing a relatively much drier surface in contact with the user than had been previously obtainable. However, contrary to expectations, it has been learned that despite the highly effective fluid transfer and fluid isolation characteristics exhibited by plastic topsheets of the type generally disclosed in the Thompson patent and their proven compatibility with the wearer's skin, many users find it psychologically undesirable to employ a material which is perceivably plastic in contact with their skin. It is believed that this user reaction is due partly to the highly regulated tapered capillary pattern on the wearer-contacting surface of the topsheet and partly to the glossy appearance of the film. Users are prone to view both of these characteristics negatively when dealing with plastic films which will contact the user's skin.

Prior art method and apparatus for embossing, vacuum forming and/or perforating plastic film have substantially precluded the elimination of such plastic characteristics exhibited by formed films. U.S. Patent 2,809,392 issued to Armstrong on October 15, 1957 discloses a prior art vacuum forming drum utilized to emboss a heat softened thermoplastic film drawn across its surface. The vacuum drum comprises a rolled hollow shell provided with a plurality of circumferential grooves which are positioned progressively closer together toward the ends of the drum. Holes about 1.6 mm in diameter are drilled through the shell at circumferentially spaced points in the grooves. The heat softened film is embossed by the application of vacuum to the interior surfaces of the drum while the film is in contact with the periphery thereof. As should be clear from the foregoing, the pattern of emboss is inherently governed by machining limitations utilized in constructing the drum.

U.S. Patent Re. 23,910 issued to Smith et al. on December 14, 1954 discloses yet another prior art method and apparatus for producing textured plastic films. The Smith et al. patent suggests the use of a suction box located beneath the surface of a woven wire mesh to draw a heat softened plastic film into conformity with the woven wire mesh. The patent further suggests that a specially patterned belt or fabric could be employed to deform the film in its own likeness by supporting the belt or fabric on the woven wire mesh. In yet another embodiment, the process is carried out utilizing the cylindrical surface of a drum. Nonetheless, the patterns which can be imparted to the film are governed by weaving limitations in the case of the woven wire and fabric and machining/punching limitations in the case of the patterned belt or drum. Furthermore, it is preferred, according to the teachings of Smith et al., that the forming belt and the forming drum be utilized to produce discrete lengths of film rather than continuous webs in order to avoid creating joint marks where the ends of the belt or the ends of the drum are joined together.

U.S. Patent 3,054,148 issued to Zimmerli on September 18, 1962 discloses a process for producing a perforated plastic sheet. The process comprises subjecting a plasticized plastic sheet or film to the action of pressure over a perforated support or moulding element. The softened plastic material is caused to flow into the perforations of the molding element to a depth which can be regulated by control of such factors as the degree of softness of the material, the direction of pressure flow and the relative thickness of the plastic sheet. The molding element preferably comprises a drum which may be made from a metal sheet having the perforated design stamped or otherwise cut from the sheet. However, in the production of simulated fabrics or woven materials, the molding element preferably comprises a woven wire mesh.

A particularly preferred method for continuously debossing and, if desired, perforating a plastic film is disclosed in the commonly assigned allowed German Patent Application No. DE—A—2 746 440. This Application, in a preferred embodiment, discloses means for forming a plastic film exhibiting a regulated pattern of tapered capillaries as generally disclosed in the aforementioned patent to Thompson, causes a ribbon of planar thermoplastic film to be forward from a supply roll, thence about a circumferentially extending portion of a rotating debossing/perforating cylinder and then downstream where the debossed and perforated film may be further processed or wound on a spool to form a roll. The debossing/perforating cylinder preferably comprises a perforated tubular member through which a plurality of independently adjustable levels of vacuum can be applied from within the cylinder to circumferentially spaced sections of the film in contact with the exterior surface of the perforated tubular member. The apparatus further causes a virtual curtain of hot air to be directed radially inwardly towards a predetermined zone of the perforated tubular member. Thus, vacuum applied from within the cylinder acts in concert with the curtain of hot air which flash heats the film sufficiently to effect debossing and perforating of the film running circumferentially about the rotating cylinder. The apparatus may further control tension in the film both upstream and downstream of the debossing cylinder at predetermined constant levels. The disclosed method for making the perforated tubular forming member preferably entails forming the member inside out by electrodepositing nickel about the exterior surfaces of a pattern cylinder having outwardly extending conical projections located about its periphery, and then turning it right side out by slitting it longitudinally, reverse rolling it into the desired tubular shape, and seaming it along the edges thus formed. From the foregoing it is clear that, even in this preferred film forming process, the particular shape or pattern imparted to the thermoplastic film on the surface of the forming cylinder is indirectly governed by limitations inherent in the machining or metal displacing processes utilized to form the pattern cylinder.

Accordingly, it is an object of the present invention to provide a plastic web exhibiting a three-dimensional pattern of embossments and/or perforations or any desired combination thereof which is independent of the machining and weaving limitations inherent in prior art forming surfaces.

It is yet another object of the present invention to provide a plastic web exhibiting a degree of surface roughness or texture to further aid in reducing the glossy appearance typically exhibited by plastic films.

It is still another object of the present invention to provide a fluid-pervious plastic web exhibiting a fiber-like appearance and tactile impression, i.e., an overall impression of softness, said web further exhibiting a fine scale three-dimensional microstructure comprising a regulated reticulum of capillary networks, preferably of steadily decreasing size, originating in and extending from one surface of said film and terminating in the form of apertures in the opposite surface thereof to promote rapid liquid transport in the direction of decreasing capillary size. As utilized herein, the term "microstructure" refers to a structure of such fine scale that its precise detail is readily perceived by the human eye only upon magnification by microscopic or other means well known in the art. The term "fiber-like", as utilized herein to describe the appearance of plastic webs of the present invention refers generally to any fine scale pattern or debossments or apertures, random or non-random, reticulated or non-reticulated, which connotes an overall appearance and impression of a woven or nonwoven fibrous web when viewed by the human eye.

Summary of the invention
According to the present invention there is provided a resilient three-dimensional web having first and second surfaces and comprised of fluid-impervious plastic material, wherein said first surface of said web exhibits a fiber-like appearance and tactile impression and has a multiplicity of apertures therein, each of said apertures being defined by a multiplicity of intersecting fiber-like elements interconnected to one another in the plane of said first surface, each of said fiber-like elements further exhibiting as a result of its formation a substantially uniform U-shaped cross-section along its length, said cross-section comprising a base portion in said plane of said first surface and a sidewall portion joined to each edge of said base portion, said sidewall portions extending generally in the direction of said second surface of said web, said intersecting sidewall portions being interconnected to one another intermediate said first and said second surfaces of said web, thus forming a three-dimensional microstructure. The second surface preferably contains a multiplicity of apertures and the side wall portions of the fiber-like elements define capillary networks of steadily decreasing size originating in and extending from the first surface of the web and terminating in the apertures in the second surface thereof to promote rapid fluid transport from said first surface to said second surface. Resilient plastic webs of the present invention have widespread application, particularly in absorbent structures such as bandages, diapers, and catemenial appliances such as sanitary napkins, tampons, and the like. Said webs, when made fluid-pervious, are particularly well suited for use as a wearer-contacting topsheet in such absorbent structures. However, the present invention is in no way limited to use as a topsheet in such absorbent structures. Its use may readily be extended to great advantage in many applications as an improved replacement for prior art fibrous webs and/or prior art plastic webs where a particular combination of previously unachievable properties or characteristics is desired in a single structure. Furthermore, plastic webs of the present invention may be used in lieu of prior art plastic webs to produce a superior end product. For example, a fiber-like plastic web of the present invention could serve as a breathable backsheet resistant to aqueous liquid passage as generally taught by U.S. Patent 3,989,867 issued to Sisson on November 2, 1976. This would require orienting the web so as to place the surface exhibiting the finer scale capillary openings in contact with the absorbent pad and the surface exhibiting the larger scale capillary openings opposite the wearer-contacting surface of the absorbent structure.

Forming surfaces of the present invention are preferably constructed by stacking a multiplicity of thin metal sheets or laminae, at least a portion of which exhibit dissimilar patterns of apertures therein, upon one another to form a three-dimensional reticulum exhibiting the particular structural features desired for the application of interest and bonding said stack of dissimilar sheets to one another at contact points to form an integral laminate structure without destroying said continuum. While said laminate structure may, of course, be utilized as a forming surface while in a planar condition, it is preferably further processed by causing the uppermost surface of said laminate structure to assume a radius of curvature greater than that of the lowermost surface of said laminate structure without causing delamination, thereby causing said laminate structure to assume a substantially tubular shape, and securing the opposing free edges of said laminate structure to one another without creating a discontinuity in either the exterior surface or the three-dimensional reticulum existing throughout the tubular member thus formed. In a particularly preferred embodiment of the present invention, the free edges of the tubular laminate structure are secured to one another by lap seaming to substantially avoid any discontinuity in the three-dimensional pattern exhibited at any point along its surface.

The laminate tubular member, which permits continuous web processing, is preferably utilized in a vacuum forming operation which is conducted in concert with a curtain of hot air which flash heats the plastic film sufficiently to effect substantial conformance to the three-dimensional pattern embodied in the tubular member. In other embodiments, the film may be preheated prior to contact with the forming surface, or the plastic material may be direct cast from an extruder die onto the forming surface. In any event, the film is preferably cooled while in contact with the tubular forming member and thereafter removed.

As will be apparent from the description contained herein, the present invention may be practiced to great advantage to produce films exhibiting continuous regulated patterns, continuous random patterns, or interrupted patterns merely by constructing the tubular forming member with the desired pattern on its periphery. Furthermore, the film processed thereon may be debossed and perforated, debossed only, perforated only, or any desired combination thereof.

Brief description of the drawings

While the specification concludes with claims particularly pointing out and distinctly claiming the present invention, it is believed the present invention will be better understood from the following description in conjunction with the accompanying drawings in which:

Figure 1 is a simplified perspective representation of an unfolded disposable diaper with portions of its components cut away;

Figure 2 is a plan view photograph enlarged approximately 27 times of a prior art non-woven fibrous topsheet of the type generally disclosed in U.S. Patent 4,041,951, as viewed from the wearer-contacting surface thereof;

Figure 3 is a plan view photograph enlarged approximately 27 times of the nonwoven fibrous topsheet shown in Figure 2, but taken from the absorbent pad contacting surface of the topsheet;

Figure 4 is a plan view photograph enlarged approximately 27 times of a prior art vacuum formed plastic web of the type generally disclosed in U.S. Patent 3,929,135, said photograph being taken from the wearer-contacting surface of the web;

Figure 5 is a plan view photograph enlarged approximately 27 times of the vacuum formed plastic web illustrated in Figure 4, but taken from the absorbent pad contacting surface of the web;

Figure 6 is a plan view photograph enlarged approximately 27 times of a fiber-like plastic web of the present invention, said fiber-like web having a three-dimensional microstructure comprising a regulated continuum of capillary networks of steadily decreasing size extending from its uppermost to its lowermost surface;

Figure 7 is a plan view photograph enlarged approximately 27 times of the lowermost surface of the web illustrated in Figure 6;

Figure 8 is a plan view photograph enlarged approximately 27 times of a planar segment of a photoetched laminate structure of the type utilized to form plastic webs of the type generally illustrated in Figures 6 and 7;

Figure 9 is a plan view photograph enlarged approximately 27 times of an individual lamina of the type utilized in the uppermost portion of the photoetched laminate structure illustrated in Figure 8;

Figure 10 is a plan view photograph enlarged approximately 27 times of yet another individual lamina of the type utilized in the intermediate portion of the photoetched laminate structure illustrated in Figure 8, the peripheral outline of each group of openings in said lamina being similar to the outline of the corresponding openings in the lamina illustrated in Figure 9, but further subdivided;

Figure 11 is an enlarged, partially exploded, perspective view of a photoetched laminate structure of the type generally illustrated in Figure 8, the laminae in the uppermost portions of said structure being exploded for clarity to illustrate the manner in which the opening patterns in each lamina are superposed upon one another to produce a unique three-dimensional reticulum of capillary networks of steadily decreasing size extending from the uppermost to the lowermost surface of the laminate;

Figure 12 is a perspective view similar to Figure 11 of an alternate pattern comprised of uniformly spaced holes of steadily decreasing diameter, said pattern being particularly suited to formation of a prior art plastic web having a pattern of the type generally illustrated in Figures 4 and 5;

Figure 13 is a perspective view of a tubular member formed by rolling a planar laminate structure of the type generally illustrated in Figure 8 to the desired radius of curvature and joining the free ends thereof to one another;

Figure 14 is an enlarged, simplified cross-sectional view taken along section line 14—14 of Figure 13 illustrating a preferred lap seaming technique for joining the free ends of the photoetched laminate structure to one another without substantially disrupting the three-dimensional pattern of the laminate structure in the area of joinder;

Figure 15 is a view similar to that of Figure 14 illustrating yet another lamp seaming technique which can be used to join the free ends of the photoetched laminate structure to one another without substantially disrupting the three-dimensional pattern in the area of joinder; and

Figure 16 is a simplified schematic illustration of method and apparatus for debossing and/or perforating a plastic film generally in accordance with the present invention.

Detailed description of the present invention

While the present invention will be described in the context of providing a fiber-like, resilient plastic web suitable for use as a topsheet on an absorbent bandage such as a disposable diaper, the present invention is in no way limited to such application. To the contrary, the present invention may be practiced to great advantage in many situations where it is desired to produce a plastic film or web exhibiting either a perforate or an imperforate three-dimensional structure having properties, characteristics, aesthetics, fineness of detail, etc., not previously obtainable due to machining and/or weaving limitations inherent in prior art processes for producing suitable film forming surfaces. The patterns created may be of any desired shape, they may be regulated or random, reticulated or non-reticulated, continuous or interrupted, perforated or unperforated or any desired combination thereof. The detailed description of a preferred structure and its use as a topsheet in a disposable diaper will allow one skilled in the art to readily adapt the invention to other devices.

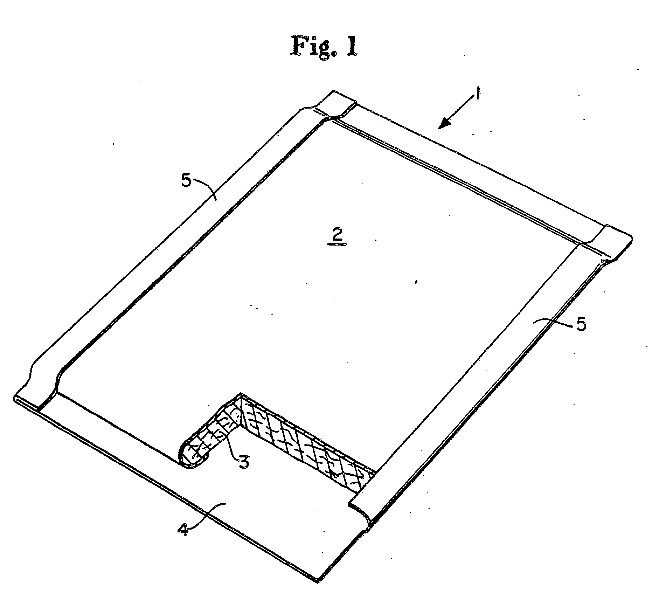

Figure 1 is a perspective view of a disposable diaper in an unfolded condition. Various layers have been cut away to more clearly show the structural details of this embodiment. The disposable diaper is referred to generally by the reference numeral 1. The fluid-pervious topsheet is shown at 2. The other two major components of the disposable diaper 1 are the absorbent element or pad 3 and the fluid-impervious backsheet 4. In general, the side flaps 5 of the backsheet 4 are folded so as to cover the edges of the absorbent pad 3 and topsheet 2. Topsheet 2 is generally folded to completely enclose the ends of the absorbent pad 3. The drawing of diaper 1 in Figure 1 is a simplified representation of a disposable diaper. A more detailed description of a preferred embodiment of a disposable diaper is contained in U.S. Patent 3,952,745 issued to Duncan on April 27, 1976.

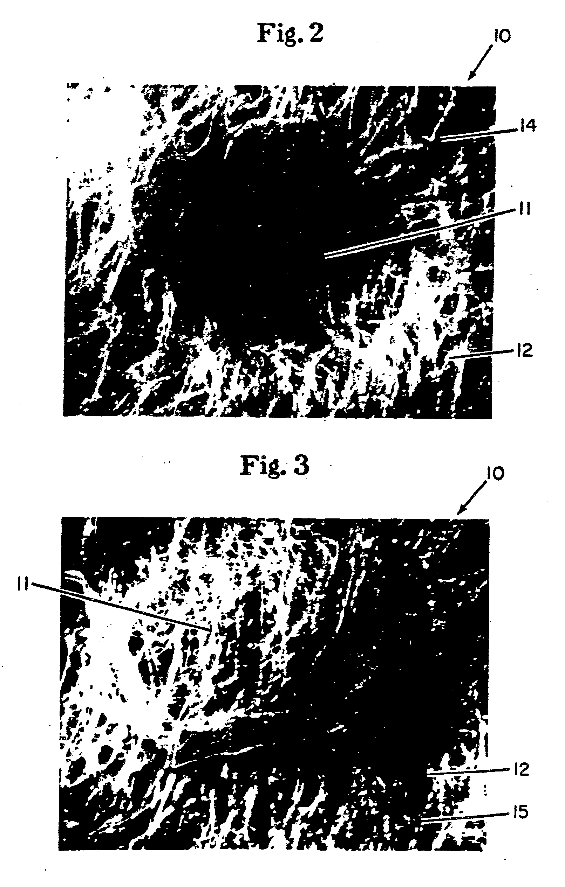

Figure 2 is a plan view photograph enlarged approximately 27 times of the wearer-contacting surface 14 of a prior art nonwoven, generally hydrophobic fibrous web 10 which has been found suitable for use as a topsheet 2 in disposable diaper 1. The web 10 preferably comprises an integral three-dimensional structure containing a multiplicity of depressed areas 11 which intimately contact the uppermost surface of the moisture absorbent pad 3, while the nondepressed areas 12 contact the wearer's skin in use. Due to the wet resilience and increased overall caliper of the nonwoven fibrous web 10, the nondepressed areas 12, which are of substantially the same density as the depressed areas 11, tend to isolate the wearer's skin from moisture contained within the moisture absorbent pad 3, thereby providing improved surface dryness and improved resistance to rewetting when the structure is temporarily subjected to pressure resulting from the wearer's body movements.

The prior art nonwoven fibrous web 10 shown in Figure 2 is generally in accordance with the teachings of U.S. Patent 4,041,951 issued to Sanford on August 16, 1977 and an object achieved by the Sanford invention is the provision of a three-dimensional topsheet which permits the free transfer of fluids discharged from the wearer's body into the absorbent element of the absorptive device and thereafter tends to isolate the wearer's skin from the fluids absorbed within the absorbent element. A further object achieved by the Sanford invention is the provision of a nonwoven fibrous topsheet which presents the user with a soft and dry nonirritating surface in contact with the skin.

Figure 3 is a plan view photograph enlarged approximately 27 times of the nonwoven fibrous web 10 illustrated in Figure 2, but taken from the lowermost or absorbent element-contacting surface 15 of the material.

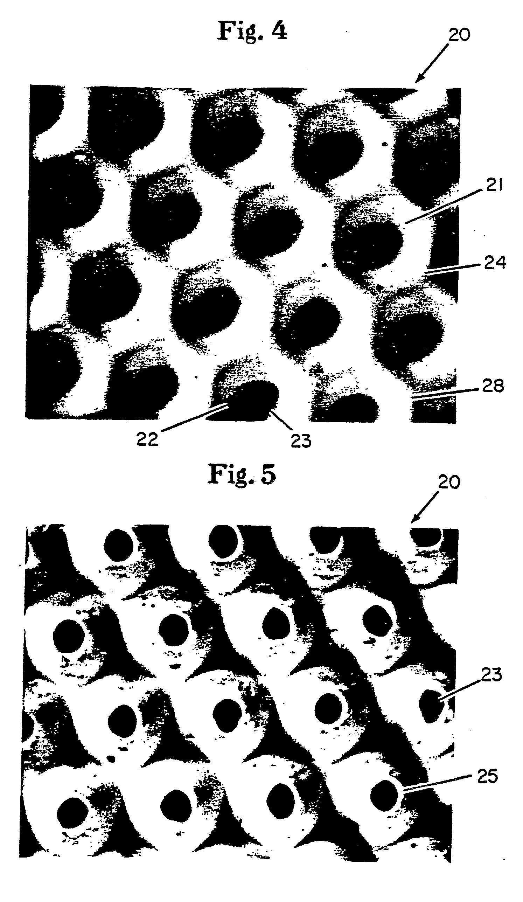

Figure 4 is a plan view photograph enlarged approximately 27 times of the wearer-contacting surface 24 of a prior art three-dimensional, fluid-pervious plastic web or film which has also been found particularly suitable as a topsheet 2 for disposable diaper 1. The fluid pervious plastic web 20 illustrated in Figure 4 exhibits a multiplicity of tapered capillaries 21 of critical diameters and tapers, each capillary having a base opening 22 in the wearer-contacting plane 28 of the web and an apex opening 23 remote from the wearer-contacting plane of the web. The fluid-pervious plastic web 20, which was vacuum formed from .0645 mm thick unembossed polyethylene, is generally in accordance with the teachings of U.S. Patent 3,929,135 issued to Thompson on December 30, 1975. The tapered capillaries 21 contained in thermoplastic web 20 of Figure 4 have a base opening 22 measuring approximately 1 mm, an apex opening 23 measuring approximately 0.4 mm, an overall height of approximately 3.0 mm and a density of approximately 97 evenly spaced apertures per square centimetre. The tapered capillaries 21 allow the free transfer of fluids from the wearer's body into the absorbent element 3 of the diaper illustrated in Figure 1 while inhibiting reverse flow of the absorbed fluids, thereby providing a relatively dry surface in contact with the wearer.

Despite the effective functioning of the prior art fluid-pervious plastic web 20 in topsheet applications for disposable absorbent bandages, it has been observed that users often psychologically resist placing a plastic material in contact with the skin. It is believed that this is due in large part to the extremely regulated nature of the pattern of capillaries 21 in the prior art plastic web 20, and also to the slickness or gloss which the user perceives in merely looking at any prior art plastic web or film. This slickness phenomenon is further illustrated in Figure 5 which is a photographic reproduction enlarged approximately 27 times of the lowermost or absorbent element-contacting surface 25 of the plastic web 20 shown in Figure 4.

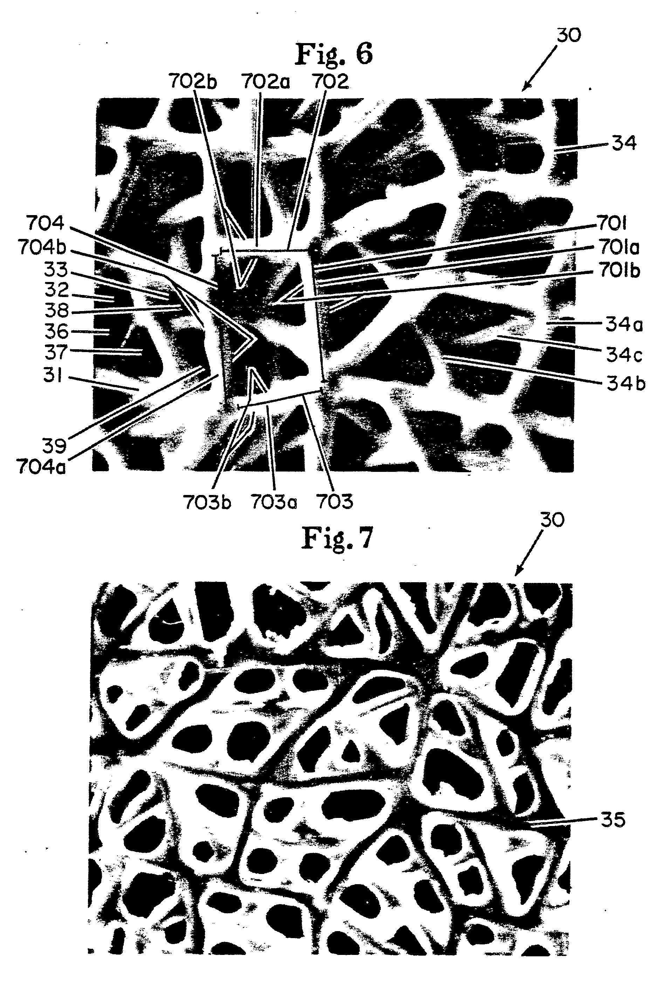

In Figure 6 is shown a plan view photograph enlarged approximately 27 times actual size of a preferred embodiment of a three-dimensional, fiber-like, fluid-pervious plastic web 30 of the present invention. The fiber-like plastic web 30 is particularly well suited for use as a diaper topsheet 2 in a disposable diaper 1 of the type generally illustrated in Figure 1. Figure 6 is an illustration of the wearing contacting or uppermost surface 34 of the web 30. The uppermost surface 34 of web 30 exhibits a fiber-like appearance by virtue of a multiplicity of intersecting fiber-like elements of which a typical group are identified as 701, 702, 703 and 704, these elements defining a series of apertures 31. The fiber-like elements 701, 702, 703, 704, are interconnected with each other in the plane 34a of the surface 34. Each element exhibits a substantially U-shaped configuration, having a base portion 701a, 702a, 703a, 704a, in the plane 34a of the uppermost surface 34 and sidewall portions 701b, 702b, 703b, 704b, joined to each edge of the base portion. The sidewall portions extend generally in the direction of the second surface 35 of the web.

In the preferred embodiment of Figure 6, the fiber-like elements of the web 30 embody a three-dimensional microstructure comprising a regulated reticulum of capillary networks of steadily decreasing size extending from the uppermost or wearer-contacting surface 34 to the lowermost or absorbent pad-contacting surface 35 thereof to promote rapid liquid transport from the uppermost to the lowermost surface of the web. A typical capillary network comprises an uppermost capillary opening 31 located in uppermost plane 34a of the web 30, said opening being further subdivided into smaller capillary openings 32 and 33 at an intermediate plane 34b. Capillary openings 32 and 33 are further subdivided into even smaller capillary openings 36, 37 and 38, 39, respectively, at lowermost plane 34c of thermoplastic web 30.

Figure 7 is a plan view photograph enlarged approximately 27 times of the fiber-like web 30 shown in Figure 6, taken from the lowermost surface 35 of the web.

From Figure 6 it should be noted that adjacent capillary networks, while exhibiting a generally similar reticulum of capillary openings of successively smaller size in the direction of the lowermost surface 35 of said web, are dissimilar in overall shape and exact manner of subdivision. Accordingly, the resultant plastic web 30 exhibits much more of a random three-dimensional fiber-like appearance and tactile impression to the user than has been obtainable by prior art methods and apparatus. The increased fluid permeability and finer detail of the fiber-like plastic web 30 of Figure 6 are clearly apparent when contrasted to a prior art plastic web 20 of the type illustrated in Figure 4.

A comparison of the wearer-contacting surface 34 of a preferred plastic web 30 of the present invention, the wearer-contacting surface 24 of the prior art plastic web 20 illustrated in Figure 4 and the wearer-contacting surface 14 of the prior art nonwoven fibrous web 10 illustrated in Figure 2 reveals a much greater similarity between the fluid-pervious plastic web 30 of the present invention and the prior art nonwoven fibrous web 10 illustrated in Figure 2 than between the prior art plastic web 20 and the nonwoven fibrous web 10. This is likewise true with respect to a comparison of the lowermost surface 35 of a plastic web 30 of the present invention, the lowermost surface 25 of the prior art plastic web 20 illustrated in Figure 5 and the lowermost surface 15 of the prior art nonwoven fibrous web 10 illustrated in Figure 3.

As should be readily apparent from the foregoing, the present invention, in a particularly preferred embodiment, combines the desirable fluid transport and anti-rewet properties provided by the tapered capillaries 21 of prior art plastic web 20 with the air permeability, wet and dry resilience, three-dimensionality, and, at least to a degree, the fiber-like feel and appearance of prior art nonwoven fibrous web 10 in a single, three-dimensional, resilient fluid-pervious plastic web 30.

To further demonstrate the improved functional characteristics exhibited by plastic webs of the present invention, samples of a prior art nonwoven web as generally shown in Figures 2 and 3 (Example I) a prior art plastic web having tapered capillaries as generally shown in Figures 4 and 5 (Example II) and a fiber-like plastic web of the present invention as generally shown in Figures 6 and 7 (Example III) were subjected to strikethrough, surface wetness, permeability and tensile strength testing.

The prior art nonwoven web (Example I) was comprised of non-woven polyester fabric such as is available from the Kendall Company of Walpole, Massachusetts. The non-woven web, which had a maximum basis weight of approximately 19.7 grams/m² exhibited a multiplicity of depressed areas as generally described in U.S. Patent 4,041,951 issued to Sanford on August 16, 1977 and incorporated herein by reference.

The prior art plastic web having tapered capillaries (Example II) was comprised of .0645 mm thick, low slip, unembossed polyethylene available from Visqueen Division of Ethyl Corporation of Baton Rouge, Lousiana under the specification Visqueen White #1850. The tapered capillaries exhibited a base opening diameter of about 1 mm, a height of about 3.0 mm, and an apex opening diameter of approximately 0.4 mm. The prior art tapered capillary film exhibited approximately 97 evenly spaced tapered capillaries per square centimetre.

The fiber-like plastic web of the present invention (Example III) was comprised of .0645 mm thick polyethylene film of the type utilized to form the web of Example II. The pattern exhibited by the Example III web was identical to that illustrated in Figures 6 and 7, which are enlarged approximately 27 times actual size.

The webs of Examples II and III were formed under similar conditions. Planar metal segments of the respective forming surfaces were preheated in a 107°C oven, and the films were heated to a temperature near their melting point prior to bringing them in contact with the forming surfaces. The lowermost sides of the respective forming surfaces were thereafter exposed to a level of vacuum sufficient to draw the films resting on their uppermost surfaces into conforming contact therewith. Perforation of the films was carried out by directing air heated to a temperature of approximately 427°C against the uppermost surfaces of the films while the lowermost surfaces of the films were exposed to vacuum on their respective forming surfaces. The films were thereafter allowed to cool on the forming surfaces and manually removed therefrom.

Several samples of each of the materials described in Examples I, II and III were thereafter subjected to comparative testing for strikethrough, surface wetness, air permeability and tensile strength. The tests conducted on the webs described in Examples I, II and III are hereinafter described in greater detail.

Strikethrough

Strikethrough, as utilized herein, is a measure of the time required for a given volume of surface-applied liquid to enter, or "strikethrough", a topsheet material into an underlying absorbent structure. In the present series of tests it is a measure of the time in seconds to completely drain 5 milliliters of simulated urine solution having a surface tension of $45 \times 10^{-5}$ Newtons/cm from a 2.5 cm diameter cavity of depth 1.6 cm having a multiplicity of holes in its lowermost surface. The cavity is integrally formed in a 10 cm×10 cm strikethrough plate which is placed on a 10 cm×10 cm composite structure comprising the topsheet being tested and an absorbent element consisting of a layer of airlaid comminuted wood pulp fibers enveloped between a pair of wet strength tissue plies. The wearer-contacting surface of the topsheet sample is oriented face-up. An electric timer is started by the simulated urine solution contacting a pair of spaced electrodes in the aforedescribed cavity. The timer automatically shuts off when all of the simulated urine solution has drained from the cavity and into the absorbent element. Times are reported in seconds.

Surface wetness

In order to compare the surface wetness characteristics of Examples I, II and III, a test designed to measure the amount of liquid which emerges from an absorbent structure, such as the disposable diaper 1 shown in Figure 1, through a topsheet to cause wetness on the surface of the topsheet was conducted. The amount of moisture drawn through the topsheet is termed "surface wetness" and serves as an estimate of how dry the wearer's skin would remain if placed in contact with the absorbent structure.

Briefly, the test comprises wetting a 10 cm by 10 cm sample of each topsheet material while superposed, wearer-contacting side up, on a standardized absorbent element preferably comprising a layer of airlaid comminuted wood pulp fibers enveloped between a pair of wet strength tissue plies with a simulated urine solution having a surface tension of approximately $45 \times 10^{-5}$ Newtons/cm until the absorbent portions of the structure, i.e., the absorbent element, including the wet strength envelope tissue has become saturated. In the present series of tests saturation did not occur until a loading factor of 4.8 had been reached, i.e., until the absorbent sample contained 4.8 grams of simulated urine solution per gram of absorbent sample. A uniform pressure loading of 3.44 kPa is applied to each sample for a period of 3 minutes so that the fluid is uniformly distributed throughout the sample. The pressure is momentarily removed, a preweighed sample of filter paper approximately 15 centimeters in diameter is inserted over the uppermost surface of the topsheet of the absorbent sample, and the predetermined pressure loading is reapplied to the sample for a period of 2 minutes. The filter paper is then removed and reweighed, and the amount of fluid absorbed by the filter paper is termed the "surface wetness" of the sample. Results are expressed in grams of fluid absorbed by the filter paper. As should thus be apparent, a lower "surface wetness" number is indicative of a dryer surface feel.

Air permeability

Air permeability of the sample webs, which is an indirect measure of breathability and comfort, was determined by placing a one inch diameter sample of each web material on a Frazier High Pressure Differential Air Permeability Tester, such as is available from Frazier Precision Instrument Company of Gaithersburg, Maryland. A Frazier No. 8 orifice plate was utilized on Examples I and III, while a No. 4 orifice plate was used on Example II to avoid blowing the fluid from the attached manometer. Results were obtained directly from the manometer and converted by means of a standardized chart to provide air flow readings in litres of air per square metre of web material per minute at 760 mm Hg, 21°C, 65% Relative Humidity, the conditions under which the tests were performed.

Tensile strength

All tensile tests were made on an Instron Model 1122 Tensile Tester, such as is available from the Instron Corporation of Canton, Massachusetts. Tests were conducted by clamping a 25 cm wide by 5 cm long sample in the tester at an initial jaw spacing of 2.5 cm. A crosshead speed of 2.5 cm per minute was applied until the sample ruptured. Readings at the point of rupture were taken from the Instron's chart recorder, and are expressed in terms of grams per cm of sample width.

Results of the aforedescribed tests reported in Table I represent the average value of all tests actually conducted for each Example. Between 2 and 5 tests were conducted for each Example.

TABLE I

| Test | Example I (Prior art non-woven fabric web as shown in Figures 2 and 3) | Example II (Prior art plastic web having tapered capillaries as shown in Figures 4 and 5) | Example III (Fiber-like plastic web of the present invention as shown in Figures 6 and 7) |
|---|---|---|---|
| Strikethrough (time in seconds) | 1.57 | 2.68 | 3.45 |
| Surface wetness (grams of simulated urine absorbed by filter paper) | 1.66 | 0.03 | 0.03 |
| Air permeability (litres of air per sq. metre material per minute at 760 mm Hg, 21°C 65% Relative Humidity) | 2212 | 487 | 1780 |
| Tensile strength/machine direction (grams per cm) | 1169 | 603 | 283 |
| Tensile strength/cross-machine direction (grams per cm) | 115 | 405 | 227 |

From the data in Table I it is clear that the directional fluid handling characteristics of the fiber-like plastic web of Example III closely approximate those exhibited by the prior art plastic web of Example II. While the performance of neither of the plastic webs is quite equal to that of the non-woven fabric web of Example I in terms of strikethrough, experience has demonstrated that the strikethrough characteristics exhibited by any of the webs considered in Examples I, II and III are quite satisfactory for use as a disposable diaper topsheet. Furthermore, it is critical to note that both plastic webs exhibit tremendous improvement over the non-woven web in terms of surface wetness performance, a characteristic which impacts significantly on wearer comfort. Hence their use as a topsheet is highly preferred in structures such as disposable diapers, sanitary napkins and the like, wherein it is desired to isolate the wearer's skin from fluids absorbed into the absorbent element of the structure.

The desirable surface wetness characteristics exhibited by the web of Example III are even more astounding when the results of the air permeability tests are compared. The air permeability of the fiber-like web of Example III is approximately 3—4 times that of the prior art tapered capillary web of Example II, and closely approaches that of the non-woven fabric web of Example I.

While the machine-direction tensile strength of the plastic web of Example III is only about one half that of the prior art plastic web of Example II and only about one fourth that of the non-woven fabric web of Example I, it is noteworthy that its cross-machine direction tensile strength is approximately double that of the non-woven fabric web of Example I. Although the web of Example III exhibits adequate tensile strength for use as a topsheet in most disposable absorbent bandage applications, the reduction in machine-direction tensile strength can, if desired, be compensated for by any of various reinforcing means well known in the art. In the case of a disposable diaper wherein the waistbands are subject to greatest tensile loading as the structure is being applied, this could be accomplished by any of various means well known in the art, i.e., reinforcing the waistband areas with beads of adhesive, leaving the waistband areas of the topsheet imperforate (this would not adversely affect the diaper's performance since body fluids are not normally discharged in this area), adding an independent reinforcing material in the waistband area, etc.

From the foregoing it is clear that plastic webs of the present invention can be made to exhibit desirable properties and characteristics which have been unachievable in a single prior art structure, i.e., the strikethrough and surface wetness characteristics of prior art plastic webs employing tapered capillary openings in conjunction with an air permeability, softness, tactile impression, and handle approaching those of prior art non-woven fabrics.

Furthermore, plastic webs of the present invention may be particularly advantageous in situations where the point of fluid entry is reasonably well defined, such as in sanitary napkins. Fiber-like webs of the present invention may, if desired, be made to exhibit the overall three-dimensional pattern of the fiber-like forming surface, but perforated only in the area where body fluids are likely to be discharged. The discharged fluids are allowed to enter the absorbent element of the structure through the perforated areas of the web, while the imperforate areas of the web serve not only to effectively contain, but also to mask or hide the fluids absorbed by the structure. Because the unsightly appearance of the absorbed body fluids is masked, the user feels more confortable in wearing the structure until its full absorptive capacity has been utilized.

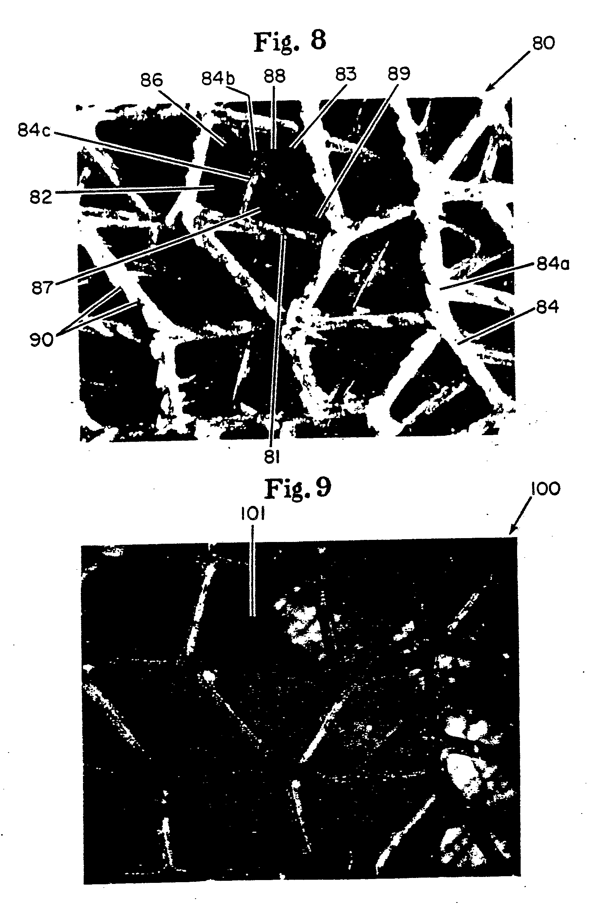

Figure 8 is a plan view photograph enlarged approximately 27 times of the film contacting surface 84 of a photoetched laminate structure 80 utilized to vacuum form an initially impervious, substantially planar, heated plastic film to produce a fluid-pervious fiber-like web 30 of the type generally illustrated in Figures 6 and 7. A comparison of Figure 8 with the fiber-like plastic web 30 shown in Figure 6 reveals the correspondence of capillary opening 31 in the uppermost plane 34a of plastic web 30 to opening 81 in the uppermost plane 84a of the photoetched laminate structure 80. Likewise, capillary openings 32 and 33 in intermediate plane 34b of plastic web 30 corresponding to intermediate openings 82 and 83, respectively, in intermediate plane 84b of photoetched laminate structure 80. Finally, capillary openings 36, 37 and 38, 39 in lowermost plane 34c of plastic web 30 correspond to openings 86, 87 and 88, 89, respectively, in lowermost plane 84c of photoetched laminate structure 80.

In the particularly preferred embodiment of the present invention shown in Figure 8, the uppermost surface of photoetched laminate structure 80 located in uppermost plane 84a is provided with a fine scale microtexture comprising a multiplicity of generally parallel V-shaped grooves 90 which help to create a random, non-planar surface appearance in the processed plastic web. The ridges and valleys formed by the V-shaped grooves 90 tend to minimize the web's gloss. This is preferably accomplished by utilizing a striated pattern exhibiting whatever shape or effect is desired in the resist coating applied to the uppermost or film-contacting surface of the lamina during the photoetching process. When the lamina is photoetched, the uncoated striations permit partial etching of the uppermost surface to form the V-shaped grooves 90 across the entire uppermost surface of the resultant photoetched lamina. As will be appreciated by those skilled in the art, any of various techniques known in the art for providing the desired degree of surface roughness may be utilized in conjunction with lamina of the present invention. While the surface roughening treatment described herein has been limited to the uppermost lamina of the structure shown in Figure 8, it may, if desired, also be applied to other lamina within the stack.

Figure 9 is a plan view photograph enlarged approximately 27 times of an individual lamina 100 of the type employed in the uppermost portion, i.e., that portion intermediate planes 84a and 84b, of the photoetched laminate structure 80 illustrated in Figure 8. As is apparent from an inspection of the photograph, opening 101 corresponds to opening 81 in laminate structure 80. Note, however, that the V-shaped grooves 90 which provide a microtexture effect in the uppermost plane 84a of the laminate structure 80 not present since the lamina 100 is not the uppermost lamina in the structure.

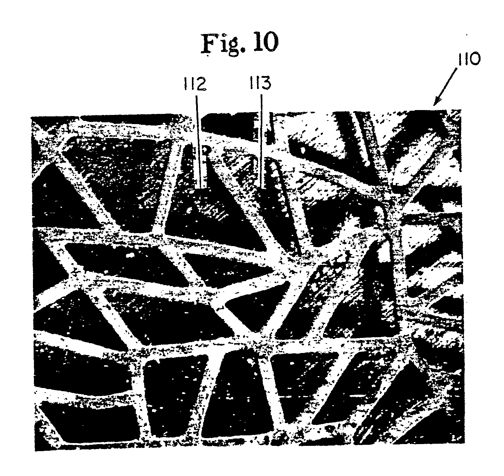

Figure 10 is a plan view photograph enlarged approximately 27 times of an individual lamina 110 of the type generally found intermediate planes 84b and 84c of the photoetched laminate structure 80 illustrated in Figure 8. Note the presence of openings 112 and 113 corresponding to openings 82 and 83 in the photoetched laminate structure 80.

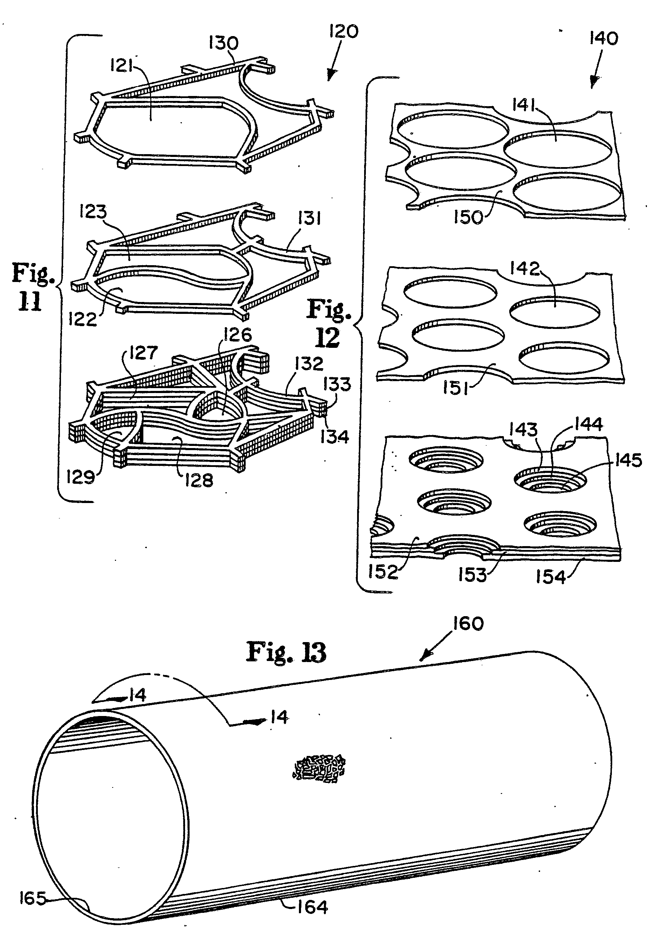

Figure 11 is a simplified, partially exploded, perspective illustration of a laminate structure 120 generally similar to that of Figure 8. The laminate structure 120 is comprised of a stack of individual lamina 130, 131, 132, 133 and 134. Each lamina has a pattern of openings therein. Lamina 132, 133 and 134 are identical to one another. In practice it is typical to employ several identical lamina superposed upon one another to provide sufficient depth of pattern in each dissimilar portion of the laminate structure. However, for simplicity of illustration a single uppermost lamina 130 and a single intermediate lamina 131 are shown. Lamina 130 exhibits a patterned arrangement of openings 121 which when superposed on lamina 131 align generally with the peripheral border formed by each pair of openings 122, 123. Similarly, the peripheral border formed by each group of openings 126, 127, 128 and 129 in lamina 132 are generally aligned with the peripheral border formed by openings 122 and 123, respectively, in lamina 131 and opening 121 in lamina 130. From the foregoing, it is readily apparent how intricate three-dimensional geometric structures can be created in nearly any pattern which is desired. It should further be apparent that the resultant three-dimensional structure, in most instances, is not susceptible of machining or weaving due to inherent limitations in the machining and weaving processes. For example, non-uniform cross-sections in which the open area is greatest near the center of the laminate structure's thickness are feasible. This can be done by employing centrally located lamina having open areas greater than those of the lamina employed for the outermost layers.

Figure 12 illustrates yet another simplified embodiment of a laminate structure 140 which could, if desired, be utilized to provide a surface suitable for debossing and perforating an initially imperforate, substantially planar plastic film to produce a fluid-pervious web exhibiting a pattern of the type generally illustrated in Figures 4 and 5. The laminate structure 140 is comprised of a stack of individual lamina 150, 151, 152, 153 and 154. Each lamina has a pattern of regularly spaced openings therein. The pattern of openings 141 in lamina 150 is concentrically aligned with the pattern of openings 142 in lamina 151, the pattern of openings 143 in lamina 152, the pattern of openings 144 in lamina 153 and the pattern of openings 145 in lamina 154. The diameter of openings 141 is greater than the diameter of openings 142 which in turn, is greater than the diameter of openings 143, etc., all the way through laminae 153 and 154. Thus the resultant laminate structure 140 provides a regulated pattern of conical openings extending from the uppermost lamina 150 through the lowermost lamina 154. If desired, the uppermost surface of lamina 150 could also be provided with a microtexture effect, i.e., V-shaped grooves as generally indicated at 90 in the embodiment of Figure 8, to provide the resultant plastic web with a microtextured effect. Thus, in addition to providing a vehicle for constructing forming surfaces which cannot be formed by prior art means, the lamination technique generally disclosed herein may be utilized as an improved vehicle for constructing forming surfaces which are capable of construction by slower, more expensive and less precise prior art means.

While photoetched and other types of laminate structures per se have for some time been generally known in the art for direct use in components such as turbines and the like, application of the lamination technique, particularly the photoetched lamination technique, in accordance with the present invention to create novel, continuous forming surfaces having unique properties and characteristics unachievable by prior art machining and/or weaving techniques is of tremendous commercial significance in that it virtually frees the plastic web forming industry from the prior art machining and/or weaving limitations inherent in prior art film forming surfaces.

One relevant prior art process for constructing a photoetched laminate structure is disclosed in Reissue Patent 29,524 reissued to Spencer on January 24, 1978. The Spencer patent discloses a method for constructing a multilayer porous material particularly suited for use in filtering and turbine cooling applications. In the disclosed embodiment, a plurality of lamina, each having a parallel slot series formed therein, is stacked with the respective adjacent slot series overlapping and extending transversely to each other. The stack is then bonded together to form a porous laminate. In a disclosed variation, the slots in one or more of the lamina are tapered to create a variable porosity in the resultant laminate structure. Basically, the disclosed process includes the steps of photoetching metal sheets to create the slot pattern, stacking a plurality of the sheets together with the slots in adjacent sheets extending transversely to each other, diffusion bonding the stacked sheets to form a porous laminate, and calendering the finished laminate structure to modify or adjust its permeability.

In a particularly preferred embodiment of the present invention, photoetched laminate structures such as the one illustrated in Figure 8 are comprised of a multiplicity of photoetched laminar stainless steel plates, each plate or each series of plates exhibiting a pattern of openings dissimilar to the adjacent plate or series of plates. The laminar plates are bonded to one another at points of contact to form an integral three-dimensional structure at whatever scale is desired for the particular application. The fineness of the pattern is of course subject to strength limitations inherent in the laminar plates. These inherent strength limitations control the minimum cross-sectional size of any structural member remaining within a given plate after the photoetching process has been carried out.

The preferred method of manufacturing laminate structures of the present invention includes the step of photoetching the desired patterns into the individual lamina. This process involves the coating of one or both sides of the lamina with a resist material in the areas to remain solid. This coating can be done in several ways well known in the art including silk screen, painting, or by photographic means. If a surface microtexture is desired in the resultant lamina, the resist coating applied to the surface may include a fine pattern of striations or the like. This is followed by a chemical etching process which dissolves or at least partially dissolves the uncoated material to thus create the individual lamina. Inasmuch as this process is known to those skilled in the art, it is not felt necessary to set forth herein a complete detailed description thereof. While photoetching, as outlined above, is generally preferred, it is of course recognized that other forming methods, such as precision stamping, could be used in some cases where the scale of reproduction and the fineness of the pattern will permit.

The photographic coating technique, which is particularly preferred in the practice of the present invention, permits creation of patterns specifically designed to provide the precise features desired in the resultant laminate, and consequently in the plastic web formed thereon, at a scale sufficiently large to be accurately drawn. The finished larger scale drawings may then be photographically reduced in size to produce the identical pattern at whatever scale or degree of fineness is desired. This photographic technique is particularly desirable in a fiber-like laminate structure of the type shown in Figure 8, wherein the reticulum of capillary networks of steadily decreasing size is created by sequential subdivision of the openings 81 originating in the uppermost plane 84a of the laminate structure 80.

The stainless steel from which the individual lamina are preferably comprised supply much of the strength necessary in the extremely fine sections of a design such as the one shown in Figure 8. The individual lamina, which are typically comprised of 410 stainless steel, may vary in thickness from 0.025 mm to 0.125 mm in any given laminate structure. Furthermore, identical lamina may be superposed upon one another to provide whatever depth or thickness is desired for any given pattern of openings within the laminate structure. To facilitate bonding, the individual lamina are preferably electroplated with a coating of pure copper which may vary in thickness from between 0.000254 mm to 0.00254 mm, depending upon the fineness of the pattern in the lamina and the desired degree of bonding in the resultant laminate structure. In a particularly preferred embodiment, individual lamina of the present invention are first cleaned and struck with a coating of nickel having a thickness on the order of 0.000254 mm to ensure more effective adherence of the copper plate to the stainless steel. While in most instances every lamina in the stack is electroplated with copper, it has been found preferable where extremely fine or delicate patterns are involved to electroplate only every other lamina in the stack to avoid an excessive buildup of copper in the resultant laminate structure.

After electroplating with copper, the individual lamina are stacked in the sequence and orientation desired to produce the resultant three-dimensional microstructure, and the stack is preferably pinned through registration holes which are photoetched in the laminar plates at the same time the pattern is etched. To avoid disruption of the three-dimensional pattern exhibited by the laminate structure, registration holes may be provided either in the borders of the desired pattern or on separate breakaway borders of the laminate structure. The pins utilized to stack the laminates are also preferably comprised of stainless steel to minimize thermal distortion, and are the exact diameter of the registration holes. A ceramic collar is preferably slipped over each pin on top and bottom of the stack of individual lamina and a stainless steel sleeve is then crimped onto the exposed portion of the pins to hold the stack tightly together in proper registration. Since the brazing operation bonds the stainless steel pins to the resultant structure, their use is generally limited to situations where they may be left in place and their ends ground off, or to situations employing break-away borders. In the event the pins must be removed after brazing, ceramic pins and ceramic collars must be employed. After pinning, the laminate stack is subjected to a furnace brazing operation to bond the stack of individual lamina into an integral structure. A honeycomb pattern, silicon nitride ceramic plate is preferably placed adjacent the uppermost and lowermost surfaces of the laminate stack, and a loading sufficient to provide good bonding but not so great as to cause distortion or deformation of the laminate stack is applied to the ceramic plates during the furnace brazing operation. To ensure the flattest possible surfaces being applied against the laminate stack, the laminate-contacting surfaces of the honeycomb plates are preferably surface ground. The honeycomb ceramic material allows uniform heating of the surfaces of the laminate structure, thereby ensuring uniform bonding throughout the contacting portions of the stack of laminar plates. One material found particularly suitable for this purpose is the silicon nitride honeycomb ceramic available from The Norton Company of Worchester, Massachusetts. While the material is available in various honeycomb sizes, 1.25 cm thick sheets having honeycomb openings measuring approximately 1.6 mm across the flats were employed on the laminate structure shown in Figure 8. An alternative material also found suitable for this purpose is a cordierite ceramic honeycomb material available from the General Refractories Company of Philadelphia, Pennsylvania.

Furnace brazing of the laminate stack to bond the individual lamina to one another is preferably carried out in a brazing furnace utilizing a pure hydrogen atmosphere to prevent oxidation of the copper. In a particularly preferred embodiment, the laminate stack assembly is placed in the furnace and heated to approximately 980°C until stabilized, i.e., until flashing of the copper plating is initiated. The furnace temperature is then elevated to approximately 1105°C and held for approximately three minutes to achieve a more normalized condition of the copper. This improves the ductility of the resultant laminate structure. Normalizing is preferably followed by rapid cooling, i.e., about 15 minutes, to approximately 93°C. The stacked laminate assembly is thereafter removed from the brazing furnace and allowed to air cool to room temperature.

It should be noted that in furnace brazing operations, it is preferable that all the lamina in a given stack be of the same grade of stainless steel and that the grain of each lamina be aligned throughout the stack to minimize the chance of distortion in the resultant laminate structure.

In photoetched laminate structures where extremely fine detail is present, a portion of the copper may tend to fillet some of the sharp corners of the three-dimensional microstructure during the furnace brazing operation. This filleting copper may be subsequently stripped out by putting the laminate assembly into a chromic acid bath for a period of time sufficient to remove the excess copper, said period of time being determined by visual observation. The stacked laminate is thereafter reinserted into the brazing furnace until it reaches a temperature of approximately 980°C and is held at that temperature for a period of about 2 minutes to even out the remaining copper plating.

In a particularly preferred embodiment of the present invention, the resultant photoetched laminate structures are rolled by conventional techniques into a tubular forming member 160, as illustrated generally in Figure 13. Contrary to expectations, it has been determined that rolling the planar laminate structure into a tubular shape does not tend to cause delamination of the structure, provided the furnace brazing operation has been properly carried out. Where extremely intricate patterns are present in the laminate structure, it has been learned that placing a thin sheet of urethane on opposite sides of the laminate structure as it is passed through the metal rolls will minimize the chance of injury to the fine pattern while rolling the member into the desired tubular shape.

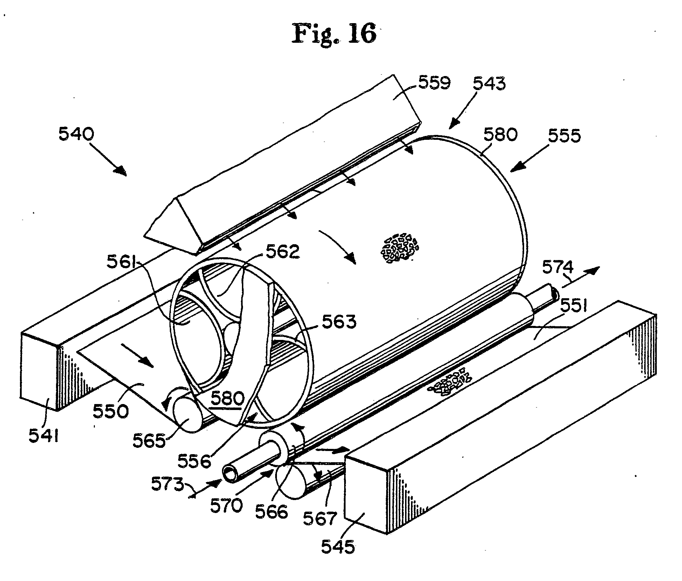

The outermost surface 164 of the tubular forming member 160 is utilized to form the plastic web brought in contact therewith while the innermost surface 165 of the tubular member generally does not contact the plastic web during the forming operation. The tubular member 160 may, in a preferred embodiment of the present invention, be employed as the forming surface on debossing/perforating cylinder 555 in a process of the type generally illustrated in Figure 16 and described in detail in the aforementioned allowed German Patent Application No. DE—A—2 746 440.

A significant advantage offered by photoetched laminate forming surfaces of the present invention when contrasted to prior art machined or woven forming surfaces is the ability to join the free ends of a single photoetched laminate section to one another or the ability to join one photoetched laminate section to another photoetched laminate section of similar pattern with substantial continuity in the three-dimensional pattern existing throughout the structure in the area of joinder. This technique may also be employed to join a multiplicity of small sections of similar photoetched laminate structures to one another where, for one reason or another, it is impractical to integrally form the individual lamina in large enough size.

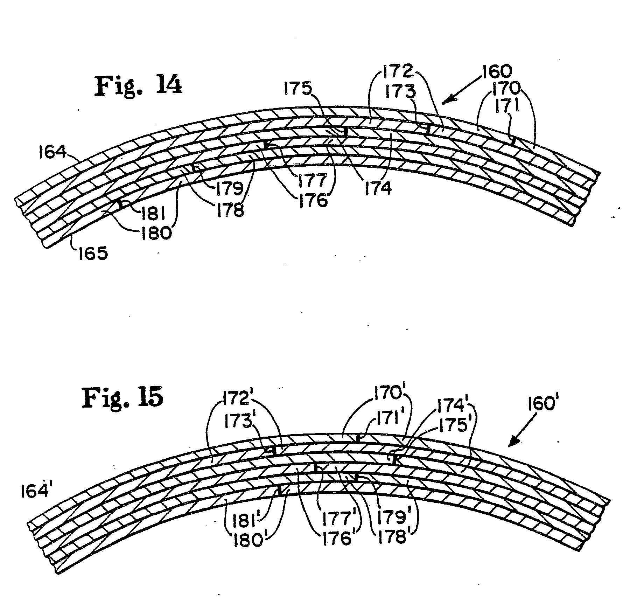

Figure 14 which is a simplified cross-sectional view taken along section line 14—14 of Figure 13 illustrates one preferred manner of joining the free ends of tubular member 160 to one another to provide an integral tubular structure exhibiting substantially no discontinuity in the three-dimensional pattern in the area of joinder. In the particularly preferred embodiment shown in Figure 14, a lap seam is created by allowing each free end of the planar photoetched laminate structure from which tubular member 160 is formed to project in a manner resembling a series of parallel stairsteps. Since the pattern exhibited by each photoetched lamina is precisely regulated and highly repeatable, rolling the planar laminate structure into a tubular shape causes the mating free ends to align with one another in stairstep fashion as illustrated in Figure 14. Thus, if the slight differences in radius of curvature of each successive lamina in the stack are ignored, corresponding parts of the pattern employed in lamina 170 mate with one another at 171; corresponding parts of the pattern employed in lamina 172 mate with one another at 173; corresponding parts of the pattern employed in lamina 174 mate with one another at 175; corresponding parts of the pattern employed in lamina 176 mate with one another at 177; corresponding parts of the pattern employed in lamina 178 mate with one another at 179; and corresponding parts of the pattern employed in lamina 180 mate with one another at 181. As is apparent from Figure 14, no individual lamina seam is radially aligned with another, yet the three-dimensional pattern of the tubular member 160 existing between the outermost surface 164 and the innermost surface 165 is substantially identical at any point along the periphery of the tubular member, including the area of joinder. Furthermore, the resultant seam has much greater strength than a radially aligned butt joint due to the reinforcing effect of one lamina on its adjacent lamina. Joinder of the lap seam shown in Figure 14 is preferably carried out by applying a low melting point, i.e., under 540°C metal bonding alloy to the area of joinder utilizing either a torch or a brazing furnace similar to that generally described in connection with lamination of the planar laminar stack. The low melting point metal bonding alloy bonds itself to the laminate structure without creating any substantial discontinuity in the area of joinder while at a temperature which is sufficiently low that it does not adversely affect the copper bonding within the laminate structure per se. Alternatively, the joint could be furnace brazed in the same manner the laminate structure is bonded together provided the areas outside the joint are protected against excessive heat.

Figure 15 is a view similar to that of Figure 14, but illustrating yet another lap seaming technique which may, if desired, be employed to join the free ends of laminar structures of the present invention to one another. Care must, however, be exercised with the construction generally illustrated in Figure 15 to prevent non-adjacent lamina from bonding to one another at their free edges during the furnace brazing operation while the laminate structure is in planar condition. One method of avoiding such problems is to temporarily insert thin ceramic paper intermediate the non-adjacent lamina at the exposed edges during the planar phase of the furnace brazing operation.

In the tubular embodiment of Figure 15, the free ends of tubular element 160′ are interleaved with one another such that, if the slight differences in radius of curvature for each successive lamina in the stack are ignored, corresponding portions of the pattern contained in lamina 170' are mated to one another at 171'; corresponding portions of the pattern contained in lamina 172' are mated to one another at 173'; corresponding portions of the pattern contained in lamina 174' are mated to one another at 175'; corresponding portions of the pattern contained in lamina 176' are mated to one another at 177'; corresponding portions of the pattern contained in lamina 178' are mated to one another at 179'; and corresponding portions of the pattern contained in lamina 180' are mated to one another at 181'. Thus, no lamina seam is in radial alignment with an adjacent lamina seam, yet the three-dimensional pattern existing between the outermost surface 164' and the innermost surface 165' of the tubular member 160' is substantially continuous at any point along the periphery of the drum, including the area of joinder of the free ends.

Thus the present invention, in addition to providing planar forming surfaces exhibiting a three-dimensional pattern unobtainable by prior art machining or weaving methods, may, if desired, be utilized to provide a cylindrical forming surface exhibiting substantial continuity of pattern along its entire periphery. This permits continuous formation of a plastic web exhibiting the desired three-dimensional pattern without a seam discontinuity of the type typically present in prior art forming surfaces. As will be readily apparent to those skilled in the art, the present invention may be applied to great advantage to produce either perforate or imperforate plastic webs exhibiting nearly any three-dimensional pattern, characteristic, property or appearance desired. The webs may be made fluid-pervious in certain areas and fluid-impervious in others by controlling the level of differential pressure applied to the film during the debossing operation.

The inherent flexibility of photographic techniques makes it feasible to create nearly any structure desired by designing the particular characteristics sought into each layer and thereafter photographically reducing or enlarging the size of the pattern to whatever scale is desired in the photoetched lamina. In other embodiments of the present invention photographs of existing structures exhibiting desirable characteristics could be utilized to form one or more of the photoetched lamina. A composite stack comprised of individual lamina of varying patterns may thereafter be assembled to produce a laminate forming surface exhibiting characteristics and properties not achievable by prior art means.

A particularly preferred continuous film forming process which may employ a tubular laminate forming surface of the type generally shown in Figures 14 and 15 is schematically illustrated in Figure 16 of DE—A—2 746 440. A particularly preferred apparatus 540 of the type disclosed in said patent application is schematically shown in Figure 16. It includes constant tension film supply means 541, debossing and perforating means 543, and constant tension film forwarding and winding means 545. The frame, bearings, supports and the like which must necessarily be provided with respect to the functional members of apparatus 540 are not shown or described in detail in order to simplify and more clearly depict and disclose the present invention, it being understood that such details would be obvious to persons of ordinary skill in the art of designing plastic film converting machinery.

Briefly, apparatus 540, Figure 16, comprises means for continuously converting a ribbon of thermoplastic film 550 into a debossed and perforated film 551 by directing hot air jets against one surface of the film while applying vacuum adjacent the opposite surface of the film, and while maintaining sufficient control of the film 550 to substantially obviate wrinkling and/or macroscopically distending the film. Thus, as will be more fully described hereinafter, apparatus 540 comprises means for maintaining constant machine direction tension in the film both upstream and downstream of a zone where the temperature is greater than the thermoplastic temperature of the film, but in which zone there is substantially zero machine direction and cross-machine direction tension tending to macroscopically distend the film. The tension is required to control and smooth a running ribbon of thermoplastic film; the zero tension zone results from the film in the zone being at a sufficiently high temperature to enable debossing, and if desired, perforating it through the use of heat and vacuum. Figure 16 also shows greatly enlarged scale perforations in film 551 to enable visually perceiving the nature of the difference between the imperforate film 550 and the debossed and perforated film 551 as more fully described hereinafter.

As can be seen in Figure 16, the debossing and perforating means 543 includes a rotatably mounted debossing-perforating cylinder 555 having closed ends 580, a nonrotating triplex vacuum manifold assembly 556 and hot air jet means 559. The triplex vacuum manifold assembly 556 comprises three manifolds designated 561, 562, and 563. Also shown in Figure 16 is a freely rotatable lead-on idler roll 565, a power rotated lead-off/chill roll 566, and a soft-face (e.g., low density neoprene) roll 567 which is driven with the chill roll. Briefly, by providing means (not shown) for independently controlling the degree of vacuum in the three vacuum manifolds, a thermoplastic ribbon of film running circumferentially about a portion of the debossing-perforating cylinder 555 is sequentially subjected to a first level of vacuum by manifold 561, a second level of vacuum by manifold 562, and a third level of vacuum by manifold 563. As will be described more fully hereinafter, the vacuum applied to the film by manifold 561 enables maintaining upstream tension in the film, vacuum applied by manifold 562 enables perforating the film when hot air is directed radially inwardly against the film, and vacuum applied by manifold 563 enables cooling the film to below its thermoplastic temperature and enables establishing downstream tension therein. If desired, the film-contacting surface of the debossing-perforating cylinder 555 may be preheated prior to reaching vacuum manifold 562 by means well known in the art (and therefore not shown) to facilitate better conformance of plastic films comprised of flow-resistant polymers during the debossing operation. The nip 570 intermediate chill roll 566 and the soft-face roll 567 is only nominally loaded because high pressure would iron-out the three-dimensional debossments which are formed in the film in the aforementioned manner. However, even nominal pressure in nip 570 helps the vacuum applied by manifold 563 to isolate downstream tension (i.e., roll winding tension) from the debossing-perforating portion of the debossing-perforating cylinder 555, and enables the nip 570 to peel the debossed and perforated film from the debossing-perforated cylinder 555. Moreover, while vacuum drawn ambient air passing through the film into manifold 563 will normally cool the film to below its thermoplastic temperature, the passage of coolant through the chill roll as indicated by arrows 573, 574 in Figure 16 will enable the apparatus to handle thicker films or be operated at higher speeds.

To summarize, the first vacuum manifold 561, and the third vacuum manifold 563 located within the debossing-perforating cylinder 555 enable maintaining substantially constant upstream and downstream tension respectively in a running ribbon of film while the intermediate portion of the film adjacent the second vacuum manifold 562 within the debossing-perforating cylinder 555 is subjected to tension vitiating heat and vacuum to effect debossing and perforating of the film.

Referring again to Figure 16, the constant tension film supply means 541 and the constant tension film forwarding the winding means 545 may, if desired, be substantially identical to and function substantially identically to the corresponding portions of the apparatus shown and described in U.S. Patent No. 3,674,221 issued to Riemersma on July 4, 1972. The debossing and perforating means 543 comprises the rotatably mounted debossing-perforating cylinder 555, means (not shown) for rotating the cylinder 555 at a controlled peripheral velocity, the non-rotating triplex vacuum manifold assembly 556 inside the debossing-perforating cylinder 555, means (not shown) for applying controlled levels of vacuum inside the three vacuum manifolds 561, 562 and 563 comprising the triplex manifold assembly 556, and hot air jet means 559.

The debossing perforating cylinder 555 may be constructed by generally following the teachings of the aforementioned DE—A— 2746440, but substituting a tubular laminate forming surface of the present invention for the perforated tubular forming surface disclosed therein.

While a preferred application of the disclosed photoetched laminate structure is in a vacuum film forming operation as generally outlined in the aforementioned DE— A2746440, it is anticipated that photoetched laminate forming structures of the present invention could be employed with equal facility to directly form a three-dimensional plastic structure of the present invention. Such a procedure would involve applying a fluid plastic material directly to the forming surface, allowing the fluid material to solidify, and thereafter removing the three-dimensional plastic structure from the forming surface. It is further anticipated that the present technology could, if desired, be incorporated in suitably reinforced film embossing rolls and the like, provided only that the embossing pressures to which the rolls will ultimately be subject are not so great as to destroy the particular three-dimensional pattern exhibited by the laminate embossing surface. A resilient back-up roll could, if desired, be utilized in such an embossing operation to avoid damaging the laminate embossing surface.

**Claims**

1. A resilient three-dimensional web (30) having first and second surfaces (34, 35) and comprised of fluid-impervious plastic material, characterised in that said first surface (34) of said web exhibits a fiber-like appearance and tactile impression and has a multiplicity of apertures (31) therein, each of said apertures (31) being defined by a multiplicity of intersecting fiber-like elements (701, 702, 703, 704) interconnected to one another in the plane (34a) of said first surface (34), each of said fiber-like elements further exhibiting as a result of its formation a substantially uniform U-shaped cross-section along its length, said cross-section comprising a base portion (701a, 702a, 703a, 704a) in said plane of said first surface and a sidewall portion joined (701b, 702b, 703b, 704b) to each edge of said base portion, said sidewall portions extending generally in the direction of said second surface (35) of said web, and the sidewall portions of intersecting fibers being interconnected to one another intermediate said first and said second surfaces of said web, thus forming a three-dimensional microstructure.

2. A resilient three-dimensional web according to claim 1, wherein said second surface (35) of said web (30) contains a multiplicity of apertures.

3. A resilient three-dimensional web according to claim 2, wherein said interconnected sidewall portions from a discrete capillary network connecting each of said apertures formed by said multiplicity of intersecting fiber-like elements in said first surface of said web with a corresponding aperture defined by said interconnected sidewall portions in said second surface of said web.

4. A resilient three-dimensional web according to claim 3, wherein said capillary networks are of non-uniform cross-section along their length.

5. A resilient web according to claim 4 characterised in that said capillary networks interconnecting said first and second surfaces of said web are of decreasing size in the direction of said second surface, thereby promoting fluid transport from said first surface to said second surface and inhibiting the flow of said fluid in the reverse direction.

6. A resilient web according to claim 5 characterised in that at least a portion of said capillary networks originating in the plane of said first surface are subdivided so as to form a multiplicity of apertures in the plane of said second surface, thereby providing a decreasing capillary size gradient in the direction of the plane of said second surface.

7. A resilient web according to any one of claims 1 to 6 characterised in that at least said first surface of said web exhibits a fine scale pattern of ridges and valleys to minimize gloss.

8. A resilient web according to any of claims 1 to 7 wherein said fluid-impervious plastic material comprises a thermoplastic film.

9. An absorbent bandage characterised in that it comprises an absorbent element for absorbing body fluids in combination with a resilient web according to any one of claims 1—8.

10. An absorbent bandage according to claim 9 wherein the resilient web forms a wearer-contacting topsheet.

11. An absorbent bandage according to either one of claims 9 and 10 wherein the resilient web forms a breathable backsheet.

12. An absorbent bandage according to any one of claims 9 to 11 in the form of a disposable diaper.

13. An absorbent bandage according to any one of claims 9 to 11 in the form of a disposable catamenial appliance.

14. A method of continuously debossing a running ribbon of substantially planar thermoplastic film to form a resilient web in accordance with any one of claims 1—13 having a three-dimensional fiber-like appearance and tactile impression characterised in that said method comprises the steps of:—

continuously bringing said film in contacting relation with a tubular-shaped perforate laminate-forming surface exhibiting a three-dimensional microstructure comprising a regulated reticulum of capillary networks of non-uniform cross-section along their length originating in and extending continuously from the film-contacting surface to the non-film-contacting surface thereof;

heating a portion of said film in contact with said forming surface above its thermoplastic temperature;

applying a sufficiently great pneumatic differential pressure to said heated thermoplastic film to cause said film to be debossed in the image of said perforate laminate-forming surface; and

cooling the debossed film below its thermoplastic temperature before removing said film from said forming surface.

15. The method of claim 14 wherein the pneumatic differential pressure applied to said heated thermoplastic film is sufficient to cause said film to be debossed and perforated in the image of said perforate laminate-forming surface.

16. The method of either one of claims 14 and 15 wherein said tubular-shaped perforate laminate-forming surface is preheated prior to bringing said film in contacting relation therewith to facilitate better conformance of said film to said forming surface.

17. The method of either one of claims 15 and 16, said method being characterised by the steps of:—

establishing and maintaining control of said film by establishing and maintaining constant tension therein;

isolating a portion of said film from said constant tension while it is in contacting relation with said forming surface; and

cooling the debossed film below its thermoplastic temperature before subjecting said film to downstream tension.

18. A method of continuously debossing a running ribbon of substantially planar plastic film to impart a three-dimensional fiber-like appearance and tactile impression thereby to form a web in accordance with any one of claims 1—13, said method being characterised by the steps of:—

continuously bringing said film in contacting relation with a tubular shaped laminate-forming surface exhibiting a three-dimensional microstructure comprising a regulated reticulum of debossed areas of non-uniform cross-section along their length, said debossed areas originating in and extending continuously from the film-contacting surface of said tubular shaped laminate-forming surface to the non-film-contacting surface thereof; and

applying sufficient mechanical pressure to said film while in contact with said forming surface to cause said film to permanently assume the three-dimensional microstructure pattern exhibited by said forming surface.

19. The method of claim 18 wherein said mechanical pressure is applied to said film by passing said film between a nip formed between said tubular shaped forming surface and a resilient back-up roll.

20. A method of continuously forming a resilient thermoplastic web in accordance with any one of claims 1—13, having a three dimensional fiber like appearance and tactile impression characterised in that said method comprises the steps of

extruding a fluid thermoplastic material to form a film in contacting relation with a tubular-shaped perforate laminate-forming surface exhibiting a three-dimensional microstructure comprising a regulated reticulum of capillary networks of non-uniform cross-section along their length originating in and extending continuously from the film-contacting surface to the non-film-contacting surface thereof;

maintaining a portion of said film in contact with said forming surface above its thermoplastic temperature;

applying a sufficiently great pneumatic differential pressure to said heated thermoplastic film to cause said film to be debossed in the image of said perforate laminate-forming surface; and

cooling the debossed film below its thermoplastic temperature before removing said film from said forming surface.

21. A method for constructing a three-dimensional tubular member for continuously imparting a three-dimensional fiber-like appearance and tactile impression to a plastic web brought into contact with its peripheral surface in accordance with the method of any one of claims 14—20, said method being characterised in that it comprises the steps of:—

(a) forming patterns of apertures in a multiplicity of planar sheets, at least a portion of said sheets having aperture patterns which are dissimilar to one another;

(b) superposing said sheets having dissimilar aperture patterns on one another to form a stack exhibiting a three-dimensional decreasing size from the uppermost surface to the lowermost surface thereof;

(c) bonding said superposed stack of sheets to one another at contact points to form an integral laminate structure without destroying said continuous pattern of capillary networks;

(d) causing the uppermost surface of said laminate structure to assume a radius of curvature greater than that of said lowermost surface of said laminate structure without causing delamination thereof, thereby causing said laminate structure to assume a substantially tubular shape; and

(e) securing the opposing free edges of said tubular shaped laminate structure to one another while maintaining substantial continuity of said three-dimensional continuous pattern of capillary networks about the entire periphery of the tubular member thus formed.

22. The method of claim 21, wherein said planar sheets are comprised of metal and said patterns of apertures are formed therein by photoetching.

23. The method of claim 22, wherein at least the uppermost planar sheet in said stack of sheets is microtextured by photoetching a fine scale pattern of grooves in its uppermost surface prior to superposing said planar sheets on one another.

24. The method of any one of claims 21 to 23 characterised in that said planar sheets are superposed to one another prior to bonding with their opposing free edges vertically misaligned with one another so that said opposing free edges of said laminate structure mate with one another when said laminate is caused to assume a substantially tubular shape to provide substantial continuity of said three-dimensional continuous pattern of capillary networks about the entire periphery of the tubular member thus formed.

25. The method of claim 24 wherein said planar sheets are so vertically misaligned when superposed on one another that the opposing free edges thereof mate with one another in stairstep fashion when said laminate structure is caused to assume a substantially tubular shape.

26. The method of claim 24 wherein said sheets are so vertically misaligned when superposed on one another that the opposing free edges thereof mate with one another in interleaved fashion when said laminate structure is caused to assume a substantially tubular shape.

**Revendications**

1. Une bande élastique à trois dimensions (30), possédant une première, et une deuxième surfaces (34, 35) et constituée d'une matériau plastique imperméable aux fluides, caractérisée en ce que la dite première surface (34) de la bande présente un aspect et une impression tactile fibreaux et possède une multitude d'ouvertures (31) aménagées dans elle, chacune de ces ouvertures (31) étant définie par une multitude d'éléments fibreux qui se coupent (701, 702, 703, 704) reliés les uns aux autres dans le plan (34a) de la première surface (34), chacun de ces éléments fibreux présentant en outre, en conséquence de sa formation, une section transversale, sur toute sa longueur, ayant la forme d'un U sensiblement uniforme, cette section transversale comprenant une partie de base (701a, 702a, 703a, 704a) dans ledit plan de ladite première surface, et une partie de paroi latérale, jointe (701b, 702b, 703b, 704b) à chaque arête de ladite partie de base, les parties de paroi latérale s'étendant généralement dans la direction de la deuxième surface (35) de la bande, et les parties de paroi latérale des fibres qui se coupent étant reliées les unes aux autres entre la première et la deuxième surfaces de la bande, formant ainsi une microstructure tridimensionnelle.

2. Une bande élastique tridimensionnelle selon la revendication 1, dans laquele la deuxième surface 35 de la bande 30 contient une multitude d'ouvertures.

3. Une bande élastique tridimensionnelle selon la revendication 2, dans laquelle les parties de paroi latérales reliées les unes aux autres forment un réseau capillaire discret reliant chacune des ouvertures formées par la multitude d'éléments fibreux qui se coupent les uns les autres dans la première surface de la bande à une ouverture correspondante définie par les parties de paroi latérale reliées les unes aux autres de la duxième surface de la bande.

4. Une bande élastique tridimensionnelle selon la revendication 3, dans laquelle les réseaux capillaires ont une section transversale non-uniforme sur leur longueur.

5. Une bande élastique selon la revendication 4, caractérisée en ce que les réseaux capillaires reliant l'une à l'autre la première et la deuxième surfaces de la bande ont une taille décroissante dans la direction de la deuxième surface, favorisant ainsi le transport des fluides entre la première surface et la deuxième surface et inhibant l'écoulement de ce fluide dans la direction inverse.

6. Une bande élastique selon la revendication 5, caractérisé en ce qu'au moins une partie des réseaux capillaires prenant naissance dans le plan de la première surface sont subdivisées de façon à former une multitude d'ouvertures dans le plan de la deuxième surface, créant ainsi un gradiant décroissant de tailles de capillaires dans la direction du plan de la deuxième surface.

7. Une bande élastique selon l'une quelconque des revendications 1 à 6, caractérisée en ce qu'au moins la première surface de la bande présente un dessin, à très fine échelle, de rides et de valées pour minimiser le brillant.

8. Une bande élastique selon l'une quelconque des revendications 1 à 7, dans laquelle le matériau plastique imperméable aux fluides comprend un film thermoplastique.

9. Un bandage absorbant, caractérisé en ce qu'il comprend un élément absorbant destiné à absorber les fluides corporels en combinaison avec une bande élastique selon l'une quelconque des revendications 1 à 8.

10. Un bandage absorbant selon la revendication 9, caractérisé en ce que la bande élastique forme une feuille endroit en contact avec le porteur.

11. Un bandage absorbant selon l'une quelconque des revendications 9 ou 10, caractérisée en ce que la bande élastique forme une feuille envers pouvant respirer.

12. Un bandage absorbant selon l'une quelconque des revendications 9 à 11, sous la forme d'une couche à jeter.

13. Un bandage absorbant selon l'une quelconque des revendications 9 à 11, sous la forme d'une garniture périodique à jeter.

14. Un procédé pour creuser en continu un ruban courant en un film thermoplastique essentiellement plan, pour former une bande élastique selon l'une quelconque des revendications 1 à 13, ayant un aspect et une impression tactile fibreux tridimensionnels, caractérisé en ce que ce procédé comprend les étapes consistant:

a) à mettre en continu le film en relation de contact avec une surface de forme tubulaire, perforée, formant un straitifié, présentant une microstructure tridimensionnelle comprenant un ensemble réticulé réglé de réseaux capillaires de section transversale non-uniform sur toute leur longueur, trouvant leur origine dans la surface en contact avec le film, et s'étendant en continu de cette surface jusqu'à sa surface qui n'est pas en contact avec le film;

b) à chauffer une partie de ce film en contact avec ladite surface de formage au-delà de sa température de thermoplasticité;

c) à appliquer une pression différentielle pneumatique suffisamment élevée au film thermoplastique chauffé pour creuser le film dans l'image de la surface perforée formant un straitifié; et

d) à refroidir le film creusé en-dessous de sa température de thermoplasticité avant d'enlever de film de la surface formante.

15. Le procédé de la revendication 14, dans lequel la pression différentielle pneumatique appliquée au film thermoplastique chauffé est suffisante pour que le film soit creusé et perforé dans l'image de la surface perforée formant le straitifié.

16. Le procédé de l'une quelconque des revendications 14 ou 15, dans lequel la surface perforée de forme tubulaire et formant un straitifié est préchauffée avant de mettre le film en relation de contact avec elle, pour faciliter une meilleur conformité du film à la surface formante.

17. Le procédé de l'une quelconque des revendications 15 ou 16, caractérisé en ce qu'il comprend les étapes consistant à créer et à maintenir un contrôle sur le film en créant et maintenant une tension constante à l'intérieur de ce dernier; à isoler une partie du film de la tension constante pendant qu'il est en relation de contact avec la surface formante; et à refroidir le film creusé en-dessous sa température de thermoplasticité avant de soumettre ce film à une tension dirigée vers le bas.

18. Un procédé de creusement continu d'un rubant courant d'un film plastique essentiellement plan pour conférer un aspect et une impression tactile fibreux et à trois dimensions, de façon à former ainsi une bande selon l'une quelconque des revendications 1 à 13, procédé caractérise en ce qu'il comprend les étapes consistant à mettre en continu le film en relation de contact avec une surface de forme tubulaire et formant un stratifié présentant une structure tridimensionnelle comprenant un ensemble rèticule régulé de zones creusées de section transversale non uniforme sur toute leur longueur, ces zones creusées prenant naissance dans la surface en contact avec le film, et s'étendant en continu à partir de cette surface en contact avec le film de la surface de forme tubulaire et formant un stratifié, pour aller vers sa surface qui n'est pas en contact avec le film; et à appliquer une pression mécanique suffisante sur le film pendant qu'il est en contact avec la surface formante pour que le film prenne d'une manière permanente la configuration de microstructure tridimensionelle présentée par ladite surface formante.

19. Le procédé de la revendication 18, dans lequel ladite pression mécanique est appliquée au film en faisant passer ce film dans l'intervalle formé entre la surface formante de forme tubulaire et un cylindre presseur élastique.

20. Un procéde de formage continue d'une bande thermoplastique élastique selon l'une quelconque des revendications 1 à 13, possédant un aspect et une impression tactile fibreux et tridimensionnels, caractérisé en ce que ce procédé comprend les étapes consistant à extruder un matériau thermoplastique fluid pour former un film en relation de contact avec une surface de forme tubulaire, perforée, formant un stratifié, présentant une microstructure tridimensionnelle, comprenant un ensemble réticulé régulier de réseaux capillaires de section transversale non uniforme le long de leur longueur, prenant naissance dans la surface en contact avec le film, et s'étendant en continu de cette surface à la surface qui n'est pas en contact avec le film; à maintenir une partie de ce film en contact avec la surface formante au-dessus de sa température de thermoplasticité; à appliquer une pression différentielle pneumatique suffisament importante au film thermoplastique chauffé pour que ce film soit creusé dans l'image de la surface perforée formant un stratifié; et à refroidir le film creusé en---dessous de sa temperature de thermoplasticité avant d'enlever ce film de la surface formante.

21. Un procédé de construction d'un élément tubulaire tridimensionnel destiné à conférer d'un manière continue un aspect et une impression tactile fibreux tridimensionnels à une bande plastique mise en contact avec sa surface périphérique conformément au procédé selon l'une quelconque des revendications 14 à 20, procédé caractérisé en ce qu'il comprend les étapes consistant (a) à former des configurations d'ouvertures dans une multitude de feuilles planes, au moins une partie de ces feuilles ayant des configurations d'ouverture différentes les unes des autres; (b) à superposer les feuilles ayant des configurations d'ouvertures différentes les unes sur les autres pour former un empilement présentant une taille tridimensionnelle décroissant entre sa surface la plus supérieure et sa surface la plus inférieure; (c) à coller cet empilement de feuilles superposées les unes aux autres en des points de contact pour former une structure stratifiée intégrale sans détruire la configuration continue de réseaux capillaires; (d) à faire en sorte que la surface la plus supérieure de la structure stratifiée prenne un rayon de courbure supérieur à celui de la surface la plus inférieure de la structure stratifiée, sans en provoquer une déstratification, faisant ainsi en sorte que la structure stratifiée prenne une forme sensiblement tubulaire; et (e) à fixer les arêtes libres opposées de la structure stratifiée de forme tubulaire les unes aux autres tout en maintenant une continuité importante des configurations continues tridimensionelles de réseaux capillaires autour de la totalité de la périphérie de l'élément tubulaire ainsi formé.

22. Le procédé de la revendication 21, dans lequel les feuilles planes sont constituées de métal et les configurations d'ouverture y sont formées par photogravure.

23. Le procédé de la revendication 22, dans lequel au moins la feuille plane la plus supérieure de l'empilement de fueilles est microtexturée par photogravure d'une configuration de rainures, à fine échelle, dans sa surface la plus supérieure, avant de superposer les feuilles planes les unes sur les autres.

24. Le procédé de l'une quelconque des revendications 21 à 23, caractérisé en ce que les feuilles planes sont superposées les unes aux autres avant d'être collées par leurs arêtes libres opposées, qui ne sont pas en alignement vertical les unes avec les autres, de façon que les arêtes libres opposées de la structure stratifiée s'adaptent les unes aux autres quand on fait en sorte que le stratifié prenne une forme sensiblement tubulaire, pour conférer une nette continuité à la configuration continue tridimensionelle de rèseaux capillaires autour de la totalité de la périphérie de l'élément tubulaire ainsi formé.

25. Le procédé de la revendication 24, dans lequel les feuilles planes sont en défaut d'alignement vertical quand elles sont superposées les unes aux autres de sorte que leurs arêtes libres opposées s'adaptent les unes aux autres comme des marches d'escalier quand on fait en sorte que la structure stratifiée prenne une forme essentiellment tubulaire.

26. Le procédé de la revendication 24, dans lequel les feuilles sont en défaut d'alignement vertical, quand elles sont superposées les unes aux autres, de sorte que leurs arêtes libres opposées s'adaptent les unes aux autres d'une façon entrelacée quand on fait en sorte que la structure stratifiée prenne une forme essentiellement tubulaire.

## Patentansprüche

1. Eine elastische dreidimensionale, ein fluidundurchlässiges Plastikmaterial umfassende Bahn (30) mit einer ersten und einer zweiten Oberfläche (34, 35), dadurch gekennzeichnet, daß die erste Oberfläche (34) der Bahn ein faserartiges Aussehen und einen faserartigen Griff hat und eine Mannigfaltigkeit von Öffnungen (31) darin aufweist, wobei jede dieser Öffnungen (31) durch eine Mannigfaltigkeit von sich überschneidenden, faserartigen Elementen (701, 702, 703, 704) begrenzt ist, die in der Ebene (34a) dieser ersten Oberfläche (34) miteinander verbunden sind, wobei jedes dieser faserartigen Elemente weiterhin, als Ergebnis seiner Enstehung, einen im wesentlichen gleichförmigen, U-förmigen Querschnitt uber seine Länge aufweist, dieser Querschnitt einen Basisteil (701a, 702a, 703a, 704) in der Ebene der ersten Oberfläche und einen mit jeder Kante des Basisteils verbundenen Seitenwandteil (701b, 702b, 703b, 704b) umfaßt diese Seitenwandteile sich im allgemeinen in Richtung auf die zweite Oberfläche (35) der Bahn hin erstrecken und die Seitenwandteile aus sich überschneidenen Fasern zwischen der ersten und zweiten Oberfläche der Bahn miteinander verbunden sind und auf diese Weise eine dreidimensionale Mikrostruktur bilden.

2. Eine elastische, dreidimensionale Bahn nach Anspruch 1, bei welcher die zweite Oberfläche (35) der Bahn (30) eine Mannigfaltigkeit von Öffnungen aufweist.

3. Eine elastische, dreidimensionale Bahn nach Anspruch 2, bei welcher die miteinander verbundenen Seitenwandteil ein diskretes Kapillarnetzwerk bilden, welches jede der durch die Mannigfaltigkeit der sich überschneidenden, faserartigen Elemente gebildeten Öffnungen in der ersten Oberfläche der Bahn mit einer entsprechenden, von miteinander verbundenen Seitenwändteilen derfinierten Öffnung in der zweiten Oberfläche der Bahn verbindet.

4. Eine elastische, dreidimensionale Bahn nach Anspruch 3, bei welcher die Kapillarnetzwerke über ihre Länge von ungleichförmigem Querschnitt sind.

5. Eine elastische Bahn nach Anspruch 4, dadurch gekennzeichnet, daß die Kapillarnetzwerke, die die erste und die zweite Oberfläche der Bahn miteinandder verbinden, eine in Richtung auf die zweite Oberfläche hin abnehmende Größe haben, wodurch eine Fluidförderung von der ersten Oberfläche zur zweiten Oberfläche hin und eine Hemmung des Fließens des Fluids in der umgekehrten Richtung begünstigt werden.

6. Eine elastische Bahn nach Anspruch 5, dadurch gekennzeichnet, daß wenigstens ein Teil der von der Ebene der ersten Oberfläche ausgehenden Kapillarnetzwerke so unterteilt ist, daß sie eine Mannigfaltigkeit von Öffnungen in der Ebene der zweiten Oberfläche bilden, wodurch in Richtung auf die Ebene der zweiten Oberfläche hin ein Gradient abnehmender Kapillargröße geschaffen wird.

7. Eine elastische Bahn nach irgendeinem der Ansprüche 1 bis 6, dadurch gekennzeichnet, daß wenigstens die erste Oberfläche der Bahn ein feingegliedertes Muster vorstehender Grate und Täler aufweist, um den Glanz auf einen Mindestwert zu bringen.

8. Eine elastische Bahn nach irgendeinem der Ansprüche 1 bis 7, bei welcher das fluidundurchlässige Plastikmaterial einen thermoplastischen Film umfaßt.

9. Ein absorbierender Verband, dadurch gekennzeichnet, daß er ein absorbierendes Element zum Absorbieren von Körperflüssigkeiten, in Kombination mit einer elastischen Bahn nach irgendeinem der Ansprüche 1 bis 8 umfaßt.

10. Ein absorbierender Verband nach Anspruch 9, bei welchem die elastische Bahn ein mit dem Träger in Berührung kommendes Deckblatt bildet.

11. Ein absorbierender Verband nach Anspruch 9 oder 10, bei welchem die elastische Bahn ein atmungsaktives Unterlagsblatt bildet.

12. Ein absorbierender Verband nach irgendeinem der Ansprüche 9 bis 11 in der Form einer Wegwerfwindel.

13. Ein absorbierender Verband nach irgendeinem der Ansprüche 9 bis 11 in der Form eines wegwerfbaren Monatshygieneartikels.

14. Ein Verfahren zum kontinuierlichen Prägen eines laufenden Bandes aus einem im wesentlichen ebenen, thermoplastischen Film unter Bildung einer elastischen Bahn nach irgendeinem der Ansprüche 1 bis 13 mit einem dreidimensionalen, faserartigen Aussehen und einem faserartigen Griff, dadurch gekennzeichnet, daß dieses Verfahren die folgenden Stufen umfaß:

ein kontinuierliches Inberührungbringen des Filmes mit einem rohrförmigen, perforierten, ein Laminat bildenden Flächenprodukt, welche eine dreidimensionale Mikrostruktur aufweist, die ein geregeltes Netz von Kapillarnetzwerken mit ungleichförmigen Querschnitt über deren Längserstreckung umfaßt, wobei diese Kapillarnetzwerke von der mit dem Film in Berührung kommenden Oberfläche ausgehen und sich kontinuierlich von der mit dem Film in Berührung kommenden Oberfläche zu der nicht mit dem Film in Berührung kommenden Oberfläche davon erstrecken;

Erhitzen eines Teils des Films in Berührung mit der formenden Oberfläche auf eine oberhalb seiner thermoplastischen Temperatur liegende Temperatur;

Anwendung eines ausreichend großen, pneumatischen Differentialdruckes auf den erhitzten, thermoplastischen Film, um eine Prägung des Films nach dem Vorbild des perforierten, ein Laminat bildenden Flächenproduktes zu bewirken; und

Abkühlen des geprägten Filmes vor der Entfernung von der formenden Oberfläche auf eine Temperatur unterhalb seiner thermoplastischen Temperatur.

15. Das Verfahren nach Anspruch 14, bei welchem der auf den erhitzten, thermoplastischen Film angewendete, pneumatische Differentialdruck ausreicht, um eine Prägung und eine Perforierung des Filmes nach dem Vorbild des perforierten, ein Laminat bildenden Flächenproduktes zu bewirken.

16. Das Verfahren nach Anspruch 14 oder 15, bei welchem das rohrförmige, perforierte, ein Laminat bildende Flächenprodukt vorerhitzt wird, bevor der Film mit ihm in Berührung gebracht wird, um eine bessere Anpassung des Filmes an die formende Oberfläche zu erleichtern.

17. Das Verfahren nach Anspruch 15 oder 16, welches Verfahren durch die folgenden Stufen gekennzeichnet ist:

Einstellen und Aufrechterhalten einer Regelung dieses Filmes durch Einstellen und Aufrechterhalten einer konstanten Spannung in demselben;

Aufhebung der konstanten Spannung in einem Teil des Filmes, während er sich in Berührung mit der formenden Oberfläche befindet; und

Abkühlen des geprägten Filmes auf eine Temperatur unterhalb seiner thermoplastischen Temperatur, bevor dieser Film einer stromabwärts gerichteten Spannung unterworfen wird.

18. Ein Verfahren zum kontinuierlichen Prägen eines laufenden Bandes aus im wesentlichen ebenem Plastikfilm, um demselben ein dreidimensionales faserartiges Aussehen und einen faserartigen Griff zu verleihen, unter Bildung einer Bahn nach irgendeinem der Ansprüche 1 bis 13, welches Verfahren durch die folgenden Stufen gekennzeichnet ist:

ein kontinuierliches Inberührungbringen des Filmes mit einem rohrförmigen, ein Laminat bildenden Flächenprodukt, welches eine dreidimensionale Mikrostruktur aufweist, die ein geregeltes Netz von geprägten Bereichen von ungleichförmigem Querschnitt über ihre Länge umfaßt, wobei die geprägten Bereiche von der mit dem Film in Berührung kommenden Oberfläche ausgehen und sich kontinuierlich von der mit dem Film in Berührung kommenden Oberfläche des rohrförmigen, ein Laminat bildenden Flächenproduktes zu der nicht mit dem Film in Berührung kommenden Oberfläche desselben erstrecken; und

Anwendung eines ausreichend großen mechanischen Druckes auf den Film, während er sich mit der formenden Oberfläche in Berührung befindet, um zur bewirken, daß der Film das von der formenden Oberfläche gezeigte, dreidimensionale Mikrostrukturmuster permanent annimmt.

19. Das Verfahren nach Anspruch 18, bei welchem dieser mechanische Druck auf den Film dadurch angewendet wird, daß derselbe durch einen Walzenspalt hindurchgeführt wird, der zwischen dem rohrförmigen, formenden Flächenprodukt und einer elastischen Gegenwalze gebildet ist.

20. Ein Verfahren zur kontinuierlichen Bildung einer elastischen, thermoplastischen Bahn nach irgendeinem der Ansprüche 1 bis 13, welche ein dreidimensionales, faserartiges Aussehen und einen faserartigen Griff aufweist, dadurch gekennzeichnet, daß dieses Verfahren die folgenden Stufen umfaßt:

Extrudieren eines fluiden, thermoplastischen Materials unter Bildung eines Filmes, der mit einem rohrformigen, perforierten, ein Laminat bildenden Flächenprodukt in Berührung gebracht wird, das eine dreidimensionale Mikrostruktur aufweist, welche Mikrostruktur ein geregeltes Netz von Kapillarnetzwerken mit nicht-gleichförmigen Querschnitt über deren Länge umfaßt, wobei diese Kapillarnetzwerke von der mit dem Film in Berührung kommenden Oberfläche ausgehen und sich kontinuierlich von der mit dem Film in Berührung kommenden Oberflache zu der nicht mit dem Film in Berührung kommenden Oberfläche des ein Laminat bildenden Flächenproduktes erstrecken;

Inberührungshalten eines Teiles des Films mit der formenden Oberfläche bei einer Temperatur oberhalb seiner thermoplastischen Temperatur;

Anwendung eines ausreichend großen, pneumatischen Differentialdruckes auf den erhitzten, thermoplastischen Film, um eine Prägung des Filmes nach dem Vorbild des perforierten, ein Laminat bildenden Flächenproduktes zu bewirken; und

Abkühlen des geprägten Films vor der Entfernung des Filmes von der formenden Oberfläche auf eine Temperatur unterhalb seiner thermoplastischen Temperatur.

21. Ein Verfahren zum Aufbauen eines dreidimensionalen, rohrförmigen Bauteils, der dazu dienen soll, einer mit seiner peripheren Oberfläche in Berührung gebrachten Plastikbahn ein dreidimensionales, faserartiges Aussehen und einen faserartigen Griff kontinuierlich zu verliehen, nach dem Verfahren nach irgendeinem der Ansprüche 14 bis 20, dadurch gekennzeichnet, daß es die folgenden Stufen umfaßt:

(a) Ausbilden von Öffnungsmustern in einer Mannigfaltigkeit von ebenen Blättern, wobei wenigstens ein Teil dieser Blätter voneinander verschiedene Öffnungsmuster aufweist;

(b) Übereinanderlegen dieser Blätter mit unterschiedlichen Öffnungsmustern unter Bildung eines Stapels, der von seiner obserten Fläche zu seiner untersten Fläche eine dreidimensionale Größenabnahme zeigt;

(c) Verbinden des Stapels aus übereinandergelegten Blättern an Berührungspunkten unter Bildung eines einstückigen Schichtgebildes ohne Zerstörung des kontinuierlichen Musters von Kapillarnetzwerken;

(d) Bewirken, daß die oberste Fläche des Schichtgebildes einen größeren Krümmungsradius annimmt als die unterste Fläche des Schichtgebildes, ohne daß dabei eine Entlaminierung des Schichttgebildes verursacht wird, wodurch bewirkt wird, daß das Schichtgebilde eine im wesentlichen rohrförmige Gestalt annimmt; und

(e) Befestigen der gegenüberliegenden, freien Kanten des rohrförmigen Schichtgebildes aneinander, wobei die Kontinuität des dreidimensionalen, kontinuierlichen Musters von Kapillarnetzwerken um den gesmaten Umfang des so gebildeten, rohrförmigen Bauteils herum im wesentlichen gewahrt bleibt.

22. Das Verfahren nach Anspruch 21, bei welchem die ebenen Blätter aus Metall bestehen und die Öffnungsmuster in denselben durch Photoätzung gebildet werden.

23. Das Verfahren nach Anspruch 22, bei welchem wenigstens dem obersten, ebenen Blatt in dem Stapel von Blättern dadurch eine Mikrotextur verliehen wird, daß in seiner obersten Fläche, vor dem Übereinanderlegen der ebenen Blätter, durch Photoätzung ein feines Muster von Vertiefungen erzeugt wird.

24. Das Verfahren nach irgendeinem der Ansprüche 21 bis 23, dadurch gekennzeichnet, daß die ebenen Blätter, bevor sie miteinander verbunden werden, so übereinandergelegt werden, daß ihre gegenüberliegenden, freien Kanten vertikal nicht aufeinander ausgerichtet sind, so daß diese gegenüberliegenden, freien Kanten des Schichtgebildes erst dann aufeinanderpassen, wenn Maßnahmen getroffen werden, die bewirken, daß das Schichtgebilde unter Erzielung von im wesentlichen Kontinuität des dreidimensionalen, kontinuierlichen Musters von Kapillarnetzwerken rund um den gesamten Umfang des so gebildeten, rohrförmigen Bauteils herum eine im wesentlichen rohrförmige Gestalt annimmt.

25. Das Verfahren nach Anspruch 24, bei welchem die ebenen Blätter beim Übereinanderlegen in eine solche vertikale Nichtausrichtung aufeinander gebracht werden, daß ihre gegenüberliegenden, freien Kanten treppenstufenartig aufeinanderpassen, wenn das Schichtgebilde durch geeignete Maßnahmen in eine im wesentlichen rohrförmige Gestalt gebracht wird.

26. Das Verfahren nach Anspruch 24, bei welchem die Blätter beim Übereinanderlegen in eine solche vertikale Nichtausrichtung aufeinander gebracht werden, daß ihre gegenüberliegenden freien Kanten zwischenblattartig aufeinanderpassen, wenn das Schichtgebilde durch geeignete Maßnahmen in eine im wesentlichen rohrförmige Gesalt gebracht wird.

## Fig. 1

## Fig. 2

## Fig. 3

## Fig. 4

## Fig. 5

## Fig. 6

702b  702a  702

704
704b
33
32
38
36
37
31

39
704a

703b  703a  703

30

34

701
701a
701b

34a
34c
34b

## Fig. 7

30

35

## Fig. 8

## Fig. 9

## Fig. 10

Fig. 11

Fig. 12

Fig. 13

Fig. 14

Fig. 15

8

# Fig. 16